# EUROPÄISCHE PATENTSCHRIFT

(11) **EP 1 267 819 B1**
(45) Veröffentlichungstag und Bekanntmachung des Hinweises auf die Patenterteilung: **06.08.2008**
(21) Anmeldenummer: 01909822.7
(22) Anmeldetag: 06.03.2001
(51) Int. Cl.: A61Q 17/04, A61K 8/49, C07D 241/44

(54) **Chinoxalinderivate, Zubereitungen diese enthaltend und Verwendung als UV-Filter**
Quinoxaline derivatives, compositions containing them und use as UV-filter
Dérivés de quinoxaline, compositions les contenant et utilisation comme filtre UV

(30) Priorität: 17.03.2000 DE 10013318
(43) Veröffentlichungstag der Anmeldung: 02.01.2003
(73) Patentinhaber: Merck Patent GmbH, 64293 Darmstadt (DE)
(72) Erfinder: PFLÜCKER, Frank, 64297 Darmstadt (DE); DRILLER, Hansjürgen, 64823 Gross-Umstadt (DE); KIRSCHBAUM, Michael, 64331 Weiterstadt (DE); SCHOLZ, Volker, 64295 Darmstadt (DE); NEUNHOEFFER, Hans, 64367 Mühltal (DE)
(86) Internationale Anmeldenummer: PCT/EP2001/002517
(87) Internationale Veröffentlichungsnummer: WO 2001/068047

(56) Entgegenhaltungen:
- EP-A- 0 728 481
- WO-A-00/61631
- WO-A-98/15276
- US-A- 3 366 668
- US-A- 4 654 339
- LORIGA M ET AL: "QUINOXALINE CHEMISTRY.PART 5" FARMACO, SOCIETA CHIMICA ITALIANA, PAVIA, IT, Bd. 51, Nr. 8/9, 1996, Seiten 559-568, XP002100498 ISSN: 0014-827X
- FORD E ET AL: "Regioselective substitution of 2,3-dichloro-6-amino-quinoxaline" TETRAHEDRON LETTERS, ELSEVIER SCIENCE PUBLISHERS, AMSTERDAM, NL, Bd. 41, Nr. 17, April 2000 (2000-04), Seiten 3197-3198, XP004196756 ISSN: 0040-4039
- BARBARA MATUSZCZAK ET AL: "Syntheses in the series of pyrazolyl-substituted quinoxalines" HETEROCYCLES, ELSEVIER SCIENCE PUBLISHERS B.V. AMSTERDAM, NL, Bd. 45, Nr. 12, 1. Dezember 1997 (1997-12-01), Seiten 2449-2462, XP002098890 ISSN: 0385-5414
- SCHOLZ V ET AL: "A STRATEGY FOR THE DEVELOPMENT OF UV-FILTERS AND CONTROL OF THEIR ABSORPTION PROPERTIES" SOFW-JOURNAL SEIFEN, OELE, FETTE, WACHSE, VERLAG FUR CHEMISCHE INDUSTRIE, H. ZIOLKOWSKY K.G. AUGSBURG, DE, Bd. 127, Nr. 4, 1. April 2001 (2001-04-01), Seiten 3-4,6,8-11, XP001005065 ISSN: 0942-7694

## Beschreibung

Die Erfindung betrifft die Verwendung von Chinoxalinderivaten als photostabile UV-Filter.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Zubereitungen soll eine Schädigung der Keratinfaser durch UV-Strahlen vermindert werden.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, dass die Haut ihre Elastizität verliert und sich Falten bilden und führen somit zu einer frühzeitigen Alterung. Manchmal kann man auch Dermatosen beobachten, und im extremen Fall kommt es zum Auftreten von Hautkrebs.

Aufgrund dieses Wissens hat sich auch im Sonnenschutz einiges geändert. Während noch vor einigen Jahren das Hauptziel in einem erythemhemmenden UV-B-Schutz bestand, wird inzwischen ein Schutz gegen UV-A-Strahlung in Sonnenschutz Zubereitungen mit eingeschlossen.

Die UV-A-Strahlung ist im Wesentlichen Auslöser für die Pigmentierung der Haut

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenläge von weniger als 400 nm gebildet. Bekannt ist auch, dass durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt. All diese Erkenntnisse lassen daher die Entwicklung effizienter Filtersubstanzen für den UV-A- und auch für den UV-B-Bereich notwendig erscheinen.

Es besteht ein wachsender Bedarf an Lichtschutzmitteln für kosmetische und pharmazeutische Zubereitungen, die vor allem als UV-A- Filter dienen können und deren Absorptionsmaxima deshalb im Bereich von ca. 320 bis 400 nm liegen sollten. Ferner besteht auch ein Bedarf an einem Breitbandschutz, also UV-A- und UV-B-Schutz, im Bereich von 280-400 nm.

Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen oder auch in Wasser, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber oft mit den bisher verfügbaren UV-A und UV-B absorbierenden Lichtschutzmitteln nur unzureichend gewährleistet.

Es gibt im Stand der Technik zwar verschiedene Ansätze, die Photostabilität von guten Lichtschutzfiltem, wie z.B. von Dibenzoylmethanen, durch Kombination mit verschiedenen UV-B-Filtern zu verbessern (FR 2 440 933), oder auch durch Zugabe bestimmter Substanzen die UV-Filter zu stabilisieren (EP 0514491), jedoch sind damit noch keine ausreichenden Lösungen vorhanden.

Weiterhin werden in den Offenlegungsschriften DE 197 46 656 und EP 0 852 137 Substanzklassen wie 4,4-Diarylbutadiene bzw. Verbindungen mit einer R⁴-NH-CR³=CR¹R²-Struktur als neue Lichtschutzfilter vorgeschlagen, welche jedoch der Nachfrage nach geeigneten Verbindungen für den UV-A- und UV-B-Bereich nicht ausreichend genügen.

Es bestand daher die Aufgabe, eine neue Strukturklasse als Lichtschutzmittel für kosmetische und pharmazeutische Zwecke zu finden, die im UV-A- und/oder UV-B-Bereich absorbieren, die photostabil sind, eine geringe Eigenfarbe, d.h. eine scharfe Bandenstruktur aufweise, eine hohe Extinktion haben und je nach Substituent in Öl oder Wasser löslich sind.

Es wurde gefunden, dass Chinoxalinderivate mit verschiedensten Resten hervorragende UV-B- und/oder UV-A- Eigenschaften besitzen und die vorstehend beschriebenen Anforderungen in hohem Maße erfülien. Die Aufgabe wurde demnach erfindungsgemäß gelöst durch Verwendung von Chinoxalinderivaten der Formeln Ik und II.

Ähnliche Chinoxalinderivate sind zwar in der EP 0 728 481 A2 beschrieben, dort werden jedoch diese bereits bekannten Verbindungen als Arzneimittel zur Behandlung von Aids und/oder HIV-Infektionen verwendet

Gegenstand der Erfindung ist daher die Verwendung von Chinoxalinderivaten der Formeln Ik und/oder II 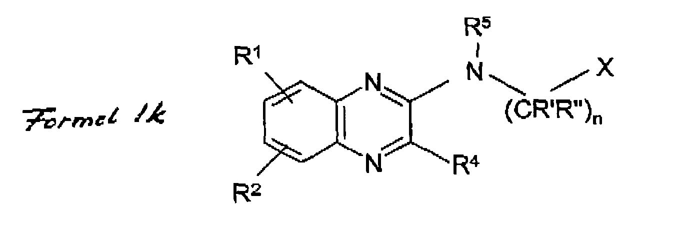 worin 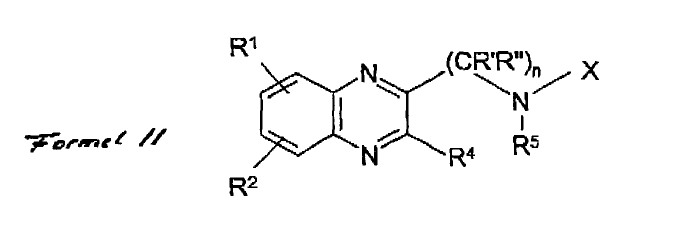
- R¹ und R²: jeweils unabhängig voneinander H, Alkyl, Alkoxy, Alkenyl oder Alkinyl jeweils mit bis zu 20 C-Atomen, Cycloalkyl, Cycloalkoxy, Cycloalkenyl oder Bicycfische Systeme jeweils mit bis zu 10 C-Atomen, wobei in allen diesen Gruppen auch ein oder mehrere Wasserstoffatome durch Sub¹ substituiert und/oder eine oder zwei CH₂-Gruppen durch C=O ersetzt sein können und in den cyclischen Systemen 1 bis 3 Heteroatome wie S, N und/oder O enthalten sein können, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶,-SO-R⁵ wasserlöslich machende Substituenten, ausgewählt aus der Gruppe bestehend aus Carboxylat-, Sulfonat- oder Ammoniumresten, COR⁵, COOR⁵, COR⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Het, -NHHet,-OHet oder -(CR⁵R⁶)ₙ-Het,
R¹ und R² zusammen auch mit Kohlenstoffatomen, an die sie gebunden sind, gemeinsam einen ungesättigten, teilweise oder vollständig gesättigten 4-, 5-, 6- oder 7-Ring bilden können, der gegebenenfalls Heteroatome wie S, N und/oder O enthalten kann, weiter anelliert und/oder auch ein- oder mehrfach substituiert sein kann,
- Sub¹: Hal, Hydroxy, Cyano, Amino, Nitro, C₁-C₄ -Alkylamino, C₁-C₄-Dialkylamino, C₁-C₄-Alkyl oder C₁-C₄ -Alkoxy, COOH oder COOAlkyl,
- Hal: Fluor, Chlor, Brom, Jod,
- n: 0, 1, 2, 3 oder 4,
- R⁵, R⁶: jeweils unabhängig voneinander H, Alkyl, Alkenyl, Alkinyl, jeweils mit bis zu 20 C-Atomen, Cycloalkyl, Cycloalkenyl, Bicyclische Systeme, jeweils mit bis zu 10 C-Atomen, wobei diese Reste bis dreifach durch Sub¹ substituiert vorliegen und/oder ein oder zwei CH₂-Gruppen durch C=O ersetzt sein können, und die cyclischen Systeme auch 1 bis 3 Heteroatome wie S, N, und/oder O enthalten können, -(CR'R")ₙ-Ar oder -(CR'R")ₙ-Het,
- R' und R": jeweils unabhängig voneinander H oder C₁-C₄-Alkyl, wobei auch ein oder zwei CH₂-Gruppen durch C=O ersetzt sein können,
- X: Ar oder Het,
- Ar: ein unsubstituierter oder ein- oder substituierter aromatischer Ring oder kondensierte Ringsysteme mit 6 bis 18 C-Atomen, worin auch ein oder zwei CH-Gruppen durch C=O ersetzt sein können,
- Het: ein unsubstituierter oder ein-oder mehrfach substituierter heteroaromatischer Ring mit 5 bis 7 Ringgliedern oder ein kondensiertes Ringsystem, wobei als Heteroatome ein oder mehrere N-, S- und/oder O-Atome enthalten sind und worin auch ein oder zwei CH-Gruppen in *α*- oder *β*-Position zu den Heteroatomen durch C=O ersetzt sein können,
- R⁴: H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome wie S, N, und/oder O enthalten können, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCC)R⁵, -(CR⁵R⁶)ₙ-NHCOOR⁵, -SR⁵, -SO₂-R⁵, NR⁵-SO-R⁶, -SO-R⁵ wasserlöslich machende Substitutenten, ausgewählt aus der Gruppe bestehend aus Carboxylat-, Sulfonat- oder Ammoniumresten, COR⁵, COOR⁵, CONR⁵R⁶, CN, O=S(-R⁵)=O, O=S(-OR⁵)=O, O=S(-NR⁵R⁶)=O, R⁷OP(-OR⁸)=O, OAr, -(CR⁵R⁶)ₙ-Ar, -Het, -NHHet,-OHet oder -(CR⁵R⁶)ₙ-Het,
- R⁷ und R⁸: jeweils unabhängig voneinander H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome wie S, N, und/oder O enthalten können,
- Sub²: Hal, Hydroxy, Cyano, Amino, Nitro, C₁-C₄-Alkyl oder C₁-C₄-Alkoxy, COR⁵, COOR⁵, OAr, OHet, -(CR⁵R⁶)ₙ-Ar oder -(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁶)=O oder R⁷OP(-OR⁸)=O,
bedeuten,
als UV-Filter substanzen.

Die Herstellung der Verbindungen gemäß Formeln Ik und/oder II kann weitgehend Literaturanalog erfolgen. Herstellfahren sind beispielsweise in
● Schipper, Day, I. An,Chem. Soc. 73(1951) 5672 und
● Miyashita, Suzuki, Iwamoto, Oishi, Higashino, Heterocycles 49 (1998) 405-413
beschrieben. Zur Herstellung von Beispielverbindungen wird insbesondere auch auf den Bespielteil dieser Anmeldung (Beispiele A - N) verwiesen,

Die Verbindungen dieser Substanzklasse der Chinoxaline zeigen hervorragende UV-absorbierende Eigenschaften sowohl im UV-A-Bereich als auch bei Vorhandensein einer zusätzlichen chormophoren Gruppe im UV-B-Bereich, wodurch ein Breitbandschutz gewährt wird. Ebenso kann leicht durch die Wahl geeigneter Substituenten die Löslichkeit der Substanzen in Wasser oder in kosmetischen Ölen induziert werden. Lipophile, d.h. die Öllöslichkeit der Verbindungen der Formeln Ik und/oder II verstärkende Rest sind beispielsweise aliphatische oder cycloaliphatische Reste, insbesondere Alkylreste mit bis zu 20 C-Atomen, Alkoxy-, Mono- und Dialkylamino-, Alkoxycarbonyl, Mono- und Dialkylaminocarbonyl, Mono- und Dialkylaminosulfonylreste, ferner auch Cyan-, Nitro-, Brom-, Chlor-, lod- oder Fluorsubstituenten.

Hydrophile, d.h. die Wasserlöslichkeit der Verbindungen der Formeln Ik und/oder II ermöglichende Reste sind z.B. Carboxy- und Sulfoxyreste und insbesondere deren Salze mit beliebigen physiologisch verträglichen Kationen, wie die Alkalisalze oder die Trikalkylammioniumsalze.

Die Alkylreste in den Resten R¹ bis R⁸ besitzen bis zu 20 C-Atomen und können unverzweigt oder verzweigt vorliegen und bedeuten demnach bevorzugt Methyl, Ethyl, n-Propyl, i-Propyl, Butyl, sek.-Butyl, i-Butyl, tert.-Butyl, Pentyl, 1-Methylbutyl, 2-Methylbutyl, 3-Methylbutyl, Hexyl, 1-Methylpentyl, 2-Methylpentyl, Heptyl, Octyl, Nonyl, Decyl, Undecyl oder Dodecyl, ferner bedeuten sie auch 2,2-Dimethylpropyl, 1-Ethylpropyl, 3-ethylpentyl, 4-Methylpentyl, 1,1-Dimethylpropyl, 1,2-Dimethylpropyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, Tridecyl, Tetradecyl, Pentadecyl, Hexadecyl, Heptadecyl, Octadecyl, Nonadecyl oder Eicosyl.

Als bevorzugte Alkenylreste seien verzweigte und unverzweigte Alkenylketten mit vorzugsweise bis zu 10 C-Atomen genannt: Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, Octenyl, Nonenyl oder Decenyl.

Als Alkinylreste kommen vorzugsweise verzweigte oder unverzweigte Alkinylketten mit bis zu 10 C-Atomen in Betracht, wie z.B. Ethinyl, Propinyl, Butinyl, i-Butinyl, Pentinyl, Hexinyl, Heptinyl oder Octinyl.

Als Cycloalkylreste seien bevorzugt verzweigte oder unverzweigte C₃-C₁₀-Cycloalkylketten wie Cyclopropyl, Cyclobutyl, Cyclopentyl, Cyclohexyl, Cycloheptyl, Cyclooctyl, 1-Methylcyclopropyl, 1,2-Dimethylcyclopentyl, 1-Methyl-2-ethylcyclopropyl, Cyclononyl oder auch Cyclodecyl genannt.

Als Alkoxyreste kommen verzweigte oder unverzweigte Alkoxyketten mit bis zu 20 C-Atomen, vorzugsweise mit bis zu 12 C-Atomen, insbesondere bevorzugt mit 1 bis 8 C-Atomen in Frage, wie z.B. Methoxy, Ethoxy, Propoxy, i-Propoxy, Butoxy, 1-Methylpropoxy, 2-Methylpropoxy, Pentoxy, 1,1-Dimethylpropoxy, 1-Methylbutoxy, 3-Methylbutoxy, 2-Methylbutoxy, Hexoxy, Heptoxy, oder auch Octoxy.

Als Cycloalkylreste seien für R¹ bis R⁸ bevorzugt verzweigte oder unverzweigte Cycloalkylketten mit 3 - 10 C-Atomen wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1,2-Dimethylcyclopropyl, 1-Methylcyclohexyl, 1,3-Dimethylcyclohexyl, Cyclooctyl, Cyclononyl oder Cyclodecyl genannt.

Als Cycloalkenylreste seien bevorzugt verzweigte oder unverzweigte C₃-C₁₀-Cycloalkenylketten mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, Cyclononenyl oder Cyclodecenyl genannt.

Als Bicycloalkyl- oder Bicycloalkenylreste seien gesättigte oder ungesättigte bicyclische Ringsysteme mit vorzugsweise bis zu 10 C-Atomen, vorzugsweise bicyclische Terpene wie Pinan-, Pinen-, Bornan-, Campherderivate, Decalin oder auch Adamantan genannt.

In diesen cyclischen Systemen können auch 1 bis 3 Heteroatome wie Schwefel, Stickstoff oder Sauerstoff enthalten sein. Beispielhaft seien hierzu Ringsysteme wie Piperidin-, Pyrrolidin-, Pyrazdin-, Morpholin-, Tetrahydro-furan-, Dihydrofuran-, Thiolan-, Piperazin-, Thiazolidin- oder auch Oxazolidingruppen genannt.

In den vor- und nachstehenden Resten, die durch Sub¹ substituiert sein können, bedeutet Sub¹ vorzugsweise Halogen, wie Fluor, Chlor, Brom oder lod, vorzugsweise Fluor oder Chlor, weiterhin bevorzugt C₁-C₄-Alkylamino oder C₁-C₄-Dialkyamino, C₁-C₄-Alkyl oder C₁-C₄-Alkoxy oder auch Hydroxy oder Amino.

In den vor- und nachstehenden Resten, die durch Sub² substituiert sein können, bedeutet Sub² vorzugsweise Halogen, wie Fluor, Chlor, Brom oder lod, vorzugsweise Fluor oder Chlor, weiterhin bevorzugt C₁-C₄-Alkylamino oder C₁-C₄-Dialkyamino, C₁-C₄-Alkyl oder C₁-C₄-Alkoxy oder auch Hydroxy oder Amino, ferner bevorzugt ist auch die Bedeutung COR⁵, -(CR⁵R⁶)ₙ-Ar, -(CR⁵R⁶)ₙ-Het, OAr, OHet, COOR⁵ oder auch R⁵OP(-OR⁶)=O.

Als Mono- oder Dialkylaminoreste kommen vorzugsweise Methylamino, Dimethylamino, Ethylamino, Methyl-ethylamino, Diethylamino, Propylamino, Methyl-propylamino, Dipropylamino, Ethyl-propylamino, Butylamino, Dibutylamino, Methyl-butylamino, Isopropylamino in Betracht, ferner auch 1,1-Dimethylpropylamino, Pentylamino, Hexylamino, 1-Methyl-1-ethylpropylamino, Heptylamino oder Octylamino.

Bevorzugt sind auch solche Verbindungen, in welchen einer der Reste R¹ und R² oder R⁴ die Bedeutung -(CR⁵R⁶)ₙ-NR⁵R⁶, -(CR⁵R⁶)ₙ-N=CR⁵R⁶ oder -(CR⁵R⁶)ₙ-CR⁵=NR⁵ besitzt. R¹ und R² können zusammen mit Kohlenstoffatomen, an die sie gebunden sind, gemeinsam einen ungesättigten, teilweise oder vollständig gesättigten 4-, 5-, 6- oder 7-Ring bilden, der gegebenenfalls Heteroatome wie S, N und/oder O enthalten kann, weiter anelliert und/oder auch ein- oder mehrfach substituiert sein kann.

In den vor- und nachstehenden Verbindungen und Formeln bedeutet Ar einen unsubstituierten oder ein- oder mehrfach substituierten aromatischen Ring oder ein kondensiertes Ringsystem mit 6 bis 18 C-Atomen, vorzugsweise mit 6 bis 10 C-Atomen, worin auch ein oder zwei CH-Gruppen durch C=O ersetzt sein können. Als insbesondere bevorzugte Gruppen seien unsubstituiertes oder substituiertes Phenyl oder Naphthyl genannt.

Het bedeutet in den vor- und nachstehenden Definitionen einen unsubstituierten oder ein- oder mehrfach substituierten heteroaromatischen Ring mit 5 bis 7 Ringgliedern oder ein kondensiertes Ringsystem mit vorzugsweise bis zu 14 Ringatomen, wobei als Heteroatome ein oder mehrere N-, S- und/oder O-Atome enthalten sind und worin auch ein oder zwei CH-Gruppen durch C=O ersetzt sein können. Soweit nicht anders definiert, haben die heterocyclischen Ringe vorzugsweise 1-13 C-Atome und 1-6 Heteratome, insbesondere 3-9 C-Atome und 1-4 Heteroatome. Beispielsweise kommen heteroaromatische Reste wie 2- oder 3-Thienyl, 2- oder 3-Furyl, 2-, 3-, oder 4-Pyridyl, Pyrimidyl, Pyrazolyl, Pyrazolonyl, Imidazolyl, Triazinyl, Pyrazinyl, Thiazolyl, Indolyl, Chinolyl, Chinoxalinyl oder Isochinolyl in Frage.

Die vorstehend beschriebenen Ar- und Het-Gruppen sind vorzugsweise unsubstituiert oder ein-, zwei- oder dreifach substituiert, wobei als Substituenten prinzipiell alle Substituenten möglich sind, solange sie keinen toxischen Eintluss auf die Gesamtverbindungen haben. Vorzugsweise kommen die als Sub² definierten Substituenten in Frage und demnach vorzugsweise folgende Substituenten: Halogen, insbesondere F oder Cl, Hydroxy, Amino, Cyano, C₁-C₄-Alkyl oder C₁-C₄ -Alkoxy, COR⁵, COOR⁵, OAr, OHet, -(CR⁵R⁶)ₙ-Ar oder -(CR⁵R⁶)ₙ-Het, -(CR⁵R⁶)ₙ-NR⁵R⁶_{,} CONR⁵R⁶, CN, O=S(-R⁵)=O, O=S(-OR⁵)=O), O=S(-NR⁵R⁶)=O oder R⁵OP(-OR⁶)=O.

R⁵ bzw. R⁶ bedeuten vorzugsweise dann H, C₁-C₄-Alkyl, Ar oder Het.

Ferner sind folgende Substituenten ganz besonders bevorzugt: Fluor, Chlor, -COOH, Alkoxy mit bis zu 8 C-Atomen, -COOAlkyl mit bis zu 8 C-Atomen, -CO-Phenyl, -CO-Aryl, -CO-Het, -chinoxalinyl, -CO-NH-R⁵. Diese Aufzählung hat rein beispielhaften Charakter und soll keineswegs limitierend sein.

In den vorstehenden Definitionen können die Gruppen Ar, Het, R⁵ und/ oder R⁶ ebenfalls wieder Substituenten, wie sie vor- oder nachstehend für diese Gruppen beschrieben sind, tragen.

Ferner sind Verbindungen der Formeln Ik und II besonders für die erfindungsgemäße Verwendung geeignet: 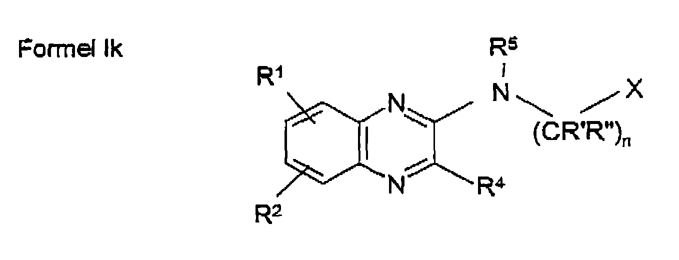 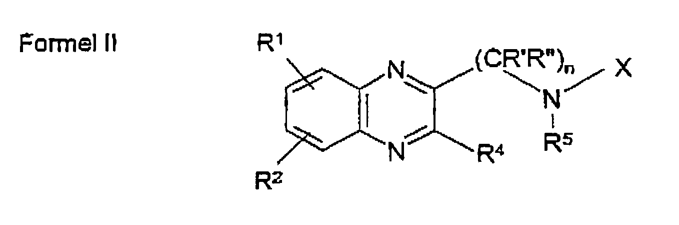 worin X einen unsubstituierten oder ein- bis dreifach substituierten aromatischen oder heteraromatischen Ring mit 5 bis 7 Ringatomen bedeutet.

Folgende Verbindungen Im bis In sind beispielsweise ebenfalls besonders bevorzugt:
- Im: Verbindungen, die ansonsten der Formel II entsprechen, worin R⁴ dann Phenyl oder -NH-Phenyl bedeuten;
- In: Verbindungen, die ansonsten der Formel Ik und/oder II entsprechen, worin R⁴ -NR⁵R⁶ ist, worin R⁵ einen Chinoxalinring und R⁶ Ar oder Het bedeutet;

Wenn die Verbindungen der Formeln Ik, II, Im, In die Gruppen Ar und/oder Het enthalten, sind diese vorzugsweise ein- bis dreifach substituiert. In Tabelle 1 werden am Beispiel des Phenylringes, welcher besonders bevorzugt gewählt wird, die bevorzugten Substituenten mit den bevorzugten Stellungen im Ring aufgelistet. Vor allem sind auch Verbindungen der Formeln Ik bis II bevorzugt, die als X einen Phenylring enthalten.

**Tabelle 1**

| R | m | Position | 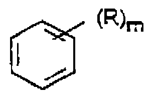 |
|---|---|---|---|
| CH₃ | 1-3 | o/m/p; m/m/p; o/p; m/m | |
| C₂H₅ | 1-3 | " | |
| C₃H₇ | 1-3 | " | |
| C₄H₉ | 1-3 | " | |
| C₅H₁₁ | 1-3 | " | |
| C₁₈H₃₇ | 1-3 | " | |
| OCH₃ | 1-3 | o/ m/ p ; m/ m/ p; o/p; m/ m | |
| OC₂H₅ | 1-3 | " | |
| OC₃H₇ | 1-3 | " | |
| OC₄H₉ | 1-3 | " | |
| OC₅H₁₁ | 1-3 | " | |
| OC₁₈H₃₇ | 1-3 | " | |
| OCOCH₃ | 1-2 | o; m; p; o/p | |
| OCOC₂H₅ | 1-2 | " | |
| OCOC₃H₇ | 1-2 | " | |
| OCOC₄H₉ | 1-2 | " | |
| OCOC₅H₁₁ | 1-2 | " | |
| OCOC₁₈H₃₇ | 1-2 | " | |
| OH | 1-3 | o/ m/ p; m/ m/ p; o/p; m/m | |
| F | 1-2 | o; p; o/p | |
| Cl | 1-2 | o; p; o/p | |
| CF₃ | 1 | o; m; p | |
| NO₂ | 1-3 | m/; m/ m; o/ o/ p | |
| NHCOR⁵ | 1 | p | |
| NHCOOR⁵ | 1 | p | |
| COR⁵ | 1-2 | o; p; o/p | |
| COOR⁵ | 1-2 | o; p; o/p | |
| CONR⁵R⁶ | 1-2 | o; p; o/p | |
| CN | 1 | p | |
| O=S(OR⁵)=O | 1 | p | |
| O=S(R⁵)=O | | p | |
| O=S(NR⁵R⁶)=O | 1 | p | |

Die Verbindungen der Formel Ik und/oder II sind größtenteils bekannt und können nach allgemein bekannten Methoden zur Herstellung von Chinoxalinderivaten (beispielsweise beschrieben in Standardwerken wie Beilstein oder Houben-Weyl) hergestellt werden. Ein Verfahren zur Herstellung von sekundären Chinoxalin-2-ylmethylaminen ist beispielweise auch beschrieben in der Patentschrift DD 281 380, worin Halogenmethylchinoxaline mit Aminoverbindungen bei erhöhter Temperatur in indifferenten Lösungsmitteln umgesetzt werden.

Die Formeln Ik und/oder II umfassen auch teilweise neue Verbindungen.

Gegenstand der Erfindung sind daher auch die neuen Verbindungen der oben angegebenen Formeln. Insbesondere sind Gegenstand die neuen Verbindungen gemäß der Formeln Ik und II, sowie die folgenden Verbindungen: 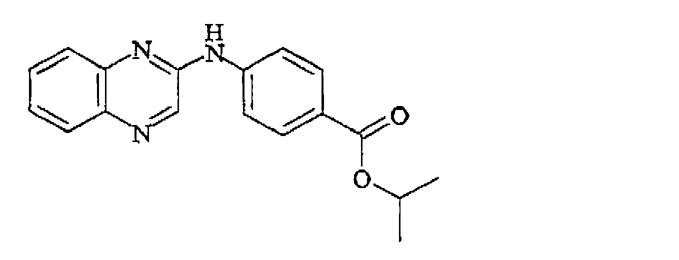
N-(2-Chinoxalinyl)-4-amino-benzoesäureisopropylester; 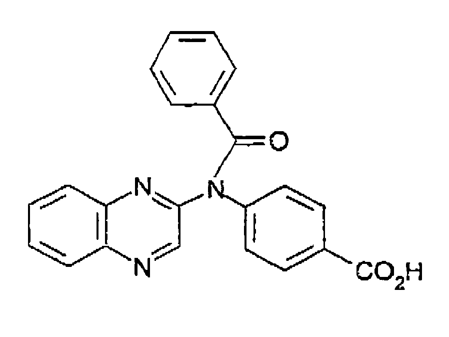
N,N-Benzoyl-(2-chinoxalinyl)-4-amino-benzoesäure; 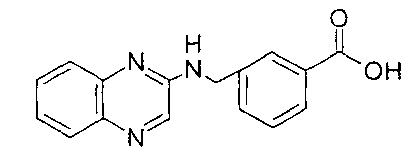
N-(2-Chinoxalinyl)-4-aminomethyl-benzoesäure; 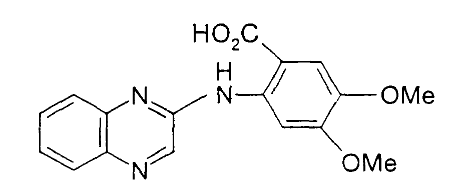
N-(2-Chinoxalinyl)-2-amino-4,5-dimethoxy-benzoesäure; 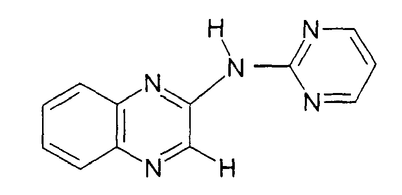
N-(2-Chinoxalinyl)-2-amino-pyrimidin; 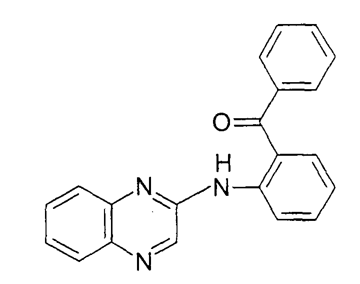
N-(2-Chinoxalinyl)-2-amino-benzophenon; 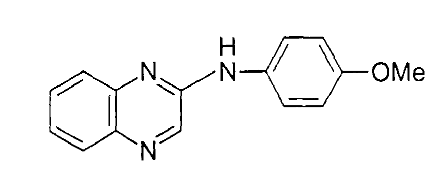
N-(2-Chinoxalinyl)-4-amino-anisol; 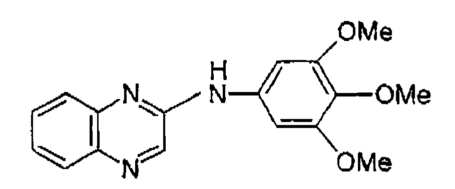
N-(2-Chinoxalinyl)-3,4,5-trimethoxy-anilin; 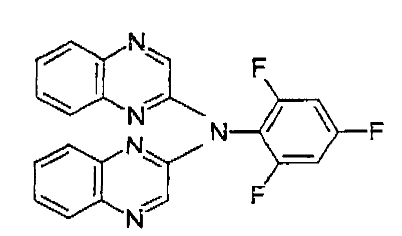
N-[Bis-(2-chinoxalinyl)]-2,4,6-trifluoro-anilin; 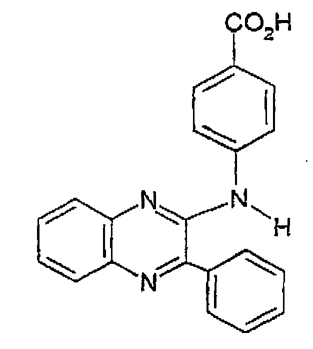
N-[2-(3-Phenyl)-chinoxalinyl]-4-amino-benzoesäure;
welche auch besonders bevorzugt verwendet werden.

Auch Triazin- bz. Aminotriazinreste sind als Substituenten am Chinoxalingerüst bevorzugt.

Folgende, bereits bekannte Chinoxalinderivate werden ebenfalls vorzugsweise für die erfindungsgemäße Verwendung eingesetzt. Diese Verbindungen sind nach literaturbekannten Methoden, die dem Fachmann geläufig sind, darstellbar.
- N-[Bis-(3-Fluoro-chinoxalin-2-yl)]-2-amino-pyrimidin
- N-(2-Chinoxalinyl)]4-amino-benzoyl-glutaminsäurediethylester
- N-(2-Chinoxalinyl)-4-amino-benzoesäure

Die erfindungsgemäßen Chinoxalinderivate sind hervorragend geeignet als UV-Filtersubstanzen. Wie schon erwähnt, können die Chinoxaline synthetisch so gestaltet werden, dass durch Vorhandensein einer zusätzlichen chromophoren Gruppe UV-absorbierende Eigenschaften sowohl im UV-A- als auch im UV-B-Bereich resultieren. Somit kann ein Breitbandschutz erzielt werden. Ferner kann durch die Wahl der Substituenten die Löslichkeit der Substanzen in Wasser oder kosmetischen Ölen beeinflusst werden.

Die Chinoxalinderivate können auch, falls gewünscht, mit beliebigen UV-Filtersubstanzen kombiniert werden, was zu einer Verbesserung der protektiven Leistung (SPF-Boost) durch synergistische Effekte führt. Im Folgenden sind einige UV-A- und UV-B-Filtersubstanzen aufgelistet, mit denen die erfindungsgemäßen Chinoxaline vorzugsweise kombiniert werden können. Diese Auswahl soll in keiner Weise limitierend sein. Die Kombination kann durch chemische Reaktion (Tabelle 2) und/oder physikalische Kombination mit UV-Filtern, die im Folgenden aufgelistet werden.

**Tabelle 2**

| 4-Aminobenzoesäure | s. Formel IV |
|---|---|
| Dimethoxyphenylglyoxalsäure | |
| 2,2'.4,4'- Tetrahydroxybenzophenon | |
| 3,3,5-Trimethyl-cyclohexyl-salicylat | |
| 2.2'-Dihydroxy-4-methoxy-benzophenon | |
| 5-Methyl-2-(1-methylethyl)-2-aminobenzoate | |
| 3-Imidazol-4-yl-acrylsäure und -ethylester | |
| 4-Isopropylbenzylsalicylat | |
| 1-(4'-Isopropylphenyl) -3-phenylpropan-1,3-dion | |
| 3-Benzyliden-bornan-2-on (3-Benzyliden-campher) | |
| 3-(4'-Methyl)benzyliden-bornan-2-on | |
| 3-(4'Trimethylammonium benzylidenbornan-2-on-methylsulfat | |
| 1-(4'-tert-Butylphenyl)-3-(4-methoxyphenyl)propan-1,3-dion (z.B. Eusolex^{®} 9020) | |
| 3-(4' Sulfo)benzyliden-bornan-2-on und Salze | |
| 2-Hydroxy-4-methoxy-benzophenon-5-sulfonsäure/ Na-Salz | |
| Salicylsäure-2-ethylhexylester | |
| 2-Hydroxy-4-methoxy-4'-methyl- benzophenon | |
| 2-Hydroxy-4-methoxy-benzophenon | |
| 4-Methoxy-zimtsäure2-isoamylester | |
| 4-Methoxy-zimtsäure2-ethylhexylester | |
| 4-Aminobenzoesäure-1-glycerylester | |

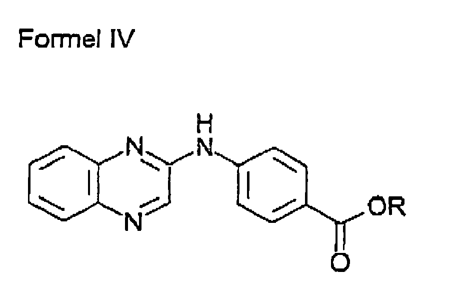

Prinzipiell kommen alle UV-Filter für eine Kombination in Frage. Besonders bevorzugt sind solche UV-Filter, deren physiologische Unbedenklichkeit bereits nachgewiesen ist. Sowohl für UV-A wie auch UV-B-Filter gibt es aus der Fachliteratur bekannte Substanzen, z.B.

Benzylidenkampferderivate wie
- 3-(4'-Methylbenzyliden)-dl-kampfer (z.B. Eusolex^{®} 6300),
- 3-Benzylidenkampfer (z.B. Mexoryl^{®} SD),
- Polymere von N-{(2 und 4)-[(2-oxobom-3-yliden)methyl]benzyl}-acrylamid (z.B. Mexoryl^{®} SW),
- N,N,N-Trimethyl-4-(2-oxobom-3-ylidenmethyl)anilinium methylsulfat (z.B. Mexoryl^{®} SK) oder
- α-(2-Oxobom-3-yliden)toluol-4-sulfonsäure (z.B. Mexoryl^{®} SL),

Benzoyl- oder Dibenzoylmethane wie
- 1-(4-tert-Butylphenyl)-3-(4-methoxyphenyl)propan-1,3-dion (z.B. Eusolex^{®} 9020) oder
- 4-isopropyldibenzoyfmethan (z.B. Eusolex^{®} 8020),
- Benzophenone wie
- 2-Hydroxy-4-methoxybenzophenon (z.B. Eusolex^{®} 4360) oder
- 2-Hydroxy-4-methoxybenzophenon-5-sulfonsäure und ihr Natriumsalz (z.B. Uvinul^{®} MS-40),
- Methoxyzimtsäureester wie
- Methoxyzimtsäureoctylester (z.B. Eusolex® 2292),
- 4-Methoxyzimtsäureisopentylester, z.B. als Gemisch der Isomere (z.B. Neo Heliopan^{®} E 1000),
- Salicylatderivate wie
- 2-Ethylhexylsalicylat (z.B. Eusolex^{®} OS),
- 4-Isopropylbenzylsalicylat (z.B. Megasol^{®}) oder
- 3,3,5-Trimethylcyclohexylsalicylat (z.B. Eusolex^{®} HMS),
- 4-Aminobenzoesäure und Derivate wie
- 4-Aminobenzoesäure,
- 4-(Dimethylamino)benzoesäure-2-ethylhexylester (z.B. Eusolex^{®} 6007),
- ethoxylierter 4-Aminobenzoesäureethylester (z.B. Uvinul^{®} P25),
und weitere Substanzen wie
- 2-Cyano-3,3-diphenylacrylsäure-2-ethylhexylester (z.B. Eusolex^{®} OCR),
- 2-Phenylbenzimidazol-5-sulfonsäure sowie ihre Kalium-, Natrium- und Triethanolaminsalze (z.B. Eusolex^{®} 232),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl^{®} SX) und
- 2,4,6-Trianilino-(p-carbo-2'-ethylhexyl-1 '-oxi)-1,3,5-triazin (z.B. Uvinul^{®} T 150).

Die in der Liste aufgeführten Verbindungen sind nur als Beispiele aufzufassen. Selbstverständlich können auch andere UV-Filter verwendet werden.
Diese organischen UV-Filter werden in der Regel in einer Menge von 0,5 bis 10 Gewichtsprozent, vorzugsweise 1-8%, in kosmetische Zubereitungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. UV-Asorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis)-1H-benzimidazol-4,6-disulfonsäure, Mononatriumsalz) (CAS-Nr. 180 898-37-7) und
- 2,2'-(1,4-Phenylen)bis)-1 H-benzimidazol-5,5'-monosulfonsäure, Mononatriumsalz
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

Diese organischen UV-Filter werden in der Regel in einer Menge von 0,5 bis 20 Gewichtsprozent, vorzugsweise 1 -15%, in kosmetische Zubereitungen eingearbeitet. In erfindungsgemäßen Formulierungen liegen sie dabei üblicherweise in Gewichtsverhältnissen von 15:1 bis 1:15, vorzugsweise von 10:1 bis 1:10 und insbesondere bevorzugt von 5:1 bis 1:5 zu den Chinoxalinderivaten der Formeln I, II oder III vor.

Bevorzugte Verbindungen mit UV-filternden Eigenschaften sind 3-(4'-Methylbenzyliden)-dl-kampfer, 1-(4-tert-Butylphenyl)-3-(4-methoxyphenyl)-propan-1,3-dion, 4-Isopropyldibenzoylmethan, 2-Hydroxy-4-methoxy-benzophenon, Methoxyzimtsäureoctylester, Methoxyzimtsäureoctylester, 3,3,5-Trimethylcyclohexylsalicytat, 4-(Dimethylamino)benzoesäure-2-ethyl-hexylester, 2-Cyano-3,3-diphenylacrylsäure-2-ethylhexylester, 2-Phenyl-benzimidazol-5-sulfonsäure sowie ihre Kalium-, Natrium- und Triethanolaminsalze.

Als anorganische UV-Filter sind beispielsweise solche aus der Gruppe der Titandioxide wie z.B. gecoatetes Titandioxid (z.B. Eusolex^{®} T-2000, Eusolex^{®}T-AQUA), Zinkoxide (z.B. Sachtotec^{®}), Eisenoxide oder auch Ceroxide denkbar. Titanoxid und Zinkoxid liegen dabei vorzugsweise als mikronisierte anorganische Pigmente vor. Diese anorganischen UV-Filter werden in der Regel in einer Menge von 0,5 bis 20 Gewichtsprozent, vorzugsweise 2 - 10 %, in kosmetische Zubereitungen eingearbeitet. In erfindungsgemäßen Formulierungen liegen sie dabei üblicherweise in Gewichtsverhältnissen von 25:1 bis 1:25, vorzugsweise von 10:1 bis 1:10 und insbesondere bevorzugt von 5:1 bis 1:5 zu den Chinoxalinderivaten der Formeln I, II oder III vor.

Die erfindungsgemäße Verwendung der Chinoxalinderivate hat einen weiteren, interessanten und vorteilhaften Aspekt. Die Kombination mit anderen UV-Filtern führt oftmals zur einer Photostabilisierung dieser anderen Substanz durch das Chinoxalinderivat.

Es ist ja bekannt, dass einige UV-Filter, die an sich vorteilhafte Lichtschutzfiltereigenschaften besitzen, den großen Nachteil einer gewissen Instabilität gegenüber UV-Strahlung haben.

Beispielsweise sind die Dibenzoylmethanderivate, wie das Eusolex 9020 (4-t-Butyl-4'-methoxy-dibenzoylmethane), solche Substanzen, die der photochemischen Zersetzung ausgesetzt sind. Die photochemischen Zersetzung dieser Verbindungsklasse folgt einer Norrish-Typ-I-Acylspaltung. Die dabei entstehenden Reaktionsprodukte stehen als Lichtschutzfiltersubstanzen nicht mehr zu Verfügung. Auch wenn schon einige Lösungsvorschläge im Stand der Technik aufgezeigt wurden, besteht dennoch stets ein Bedarf an einfachen und effektiven Wegen, dieser photolytischen Zersetzung wirksam zu begegnen.

Dafür eignen sich die hier beschriebenen Chinoxalinderivate auf hervorragende Weise, insbesondere die Kombination mit Eusolex 9020 führt zu einer stark verbesserten Photostabilisierung der Substanz.

Erfindungsgemäß können die hier beschriebenen UV-Lichtschutzfilter jeweils für sich allein oder natürlich auch in Kombination, was bevorzugt ist, in Sonnenschutzmitteln verwendet werden. Sie können mit UV-B/A-Chromophoren, z.B. allen weltweit zugelassenen und bekannten Filtern kombiniert werden zur Verbesserung der protektiven Leistung (SPF-Boost) durch synergistische Effekte. Sie sind vorzugsweise in Kombination sowohl mit anorganischen als auch mit organischen UV-A- und UV-B-Filtern oder deren Gemischen einsetzbar.

Durch Kombination von einer oder mehrerer Verbindungen der Formel Ik und/oder II mit weiteren UV-Filtem kann die Schutzwirkung gegen schädliche Einwirkungen der UV-Strahlung optimiert werden.

Ferner ist es möglich und vorteilhaft, die erfindungsgemäßen Zubereitungen mit Antioxidationsmitteln zu kombinieren. Eine solche Kombination zeigt dann sowohl Schutzwirkung als Antioxidationsmittel als auch vor Verbrennungen durch UV-Strahlung. Somit kann man auch eine schützende Wirkung gegen oxidativen Stress bzw. gegen die Einwirkung von Radikalen erzielen.

Gegenstand der Erfindung ist daher auch die Verwendung einer Verbindung der Formel Ik und/oder II gemäß Anspruch 1 in Kombination mit Antioxidationsmitteln in kosmetischen oder pharmazeutischen Zubereitungen.

Gegenstand der vorliegenden Erfindung sind daher auch kosmetische und pharmazeutische Zubereitungen, die eine oder mehrere der Verbindungen der Formeln Ik und/oder II, gegebenenfalls in Kombination mit weiteren Lichtschutzmitteln oder Antioxidationsmitteln, enthalten.

Gegenstand der Erfindung ist auch ein nicht-therapeutisches Verfahren zum Schutz der Haut und natürliche oder sensibilisierter Haare vor Sonnenstrahlen, wobei auf die Haut oder die Haare eine wirksame Menge mindestens einer Verbindung der Formel Ik und/oder II in einer kosmetische Zubereitung aufgetragen wird. Mit "sensibilisierten Haaren" sind Haare gemeint, welche einer Dauerwellenbehandlung, einem Färbe- oder Entfärbeprozess unterzogen worden sind.

Die erfindungsgemäßen Filter zum Schutz vor UV-A und UV-B-Strahlung können jeweils in Konzentrationen von 0,1 bis 20 Gew. %, vorzugsweise 1 bis 15 Gew.-%, in kosmetische Zubereitungen eingearbeitet werden. Es ist auf diese Weise möglich Zubereitungen herzustellen, in denen bis zu 100 % der eingesetzten Lichtschutzfilter die hier beschriebenen UV-Filter sind. Es handelt sich hierbei um Substanzen, die in Wasser und Ölen, je nach Substituenten am Gerüst, in einfacher Weise gelöst, dispergiert oder emulgiert werden.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime, sowie Coumaranonderivate.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 155-139), Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel eingesetzt, 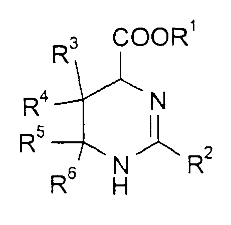 worin R¹ ein Rest H oder C1-8-Alkyl, R² ein Rest H oder C1-4-Alkyl und R³, R ⁴ R⁵ sowie R⁶ jeweils unabhängig voneinander ein Rest aus der Gruppe H, OH, NH₂ und C1-4-Alkyl sind. Bevorzugt werden Pyrimidincarbonsäuren eingesetzt, bei denen R² eine Methyl- oder eine Ethylgruppe ist und R¹ bzw. R⁵ und R⁶ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Unter Coumaranonderivaten, die in erfindungsgemäßen Zubereitungen vorteilhaft enthalten sein können, werden Verbindungen der Formel 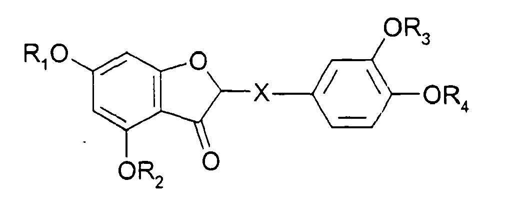 wobei -X- steht für eine Einfachbindung, -CH₂-, =CH-, -C(O)-, =C(OR⁵)-, - C(NR⁵)-, -CH(NR⁵R⁶)-, -CH(OR⁵)- und R¹, R², R³, R⁴, R⁵, und R⁶ gleich oder verschieden sein können, und unabhängig voneinander stehen für
- H
- geradkettige oder verzweigte C₁- bis C₁₂-Alkyl und/oder Alkylcarbonylgruppen,
- geradkettige oder verzweigte C₃- bis C₁₂-Alkenyl und/oder - Alkenylcarbonylgruppen,
- geradkettige oder verzweigte C₁- bis C₁₂-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- C₃- bis C₁₀-Cycloalkyl- und/oder Cycloalkylcarbonylgruppen und C₃- bis C₁₂-Cycloalkenyl- und/oder Cycloalkenylcarbonylgruppen, wobei die Ringe jeweils auch durch -(CH₂)ₙ-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste, 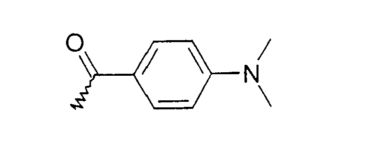 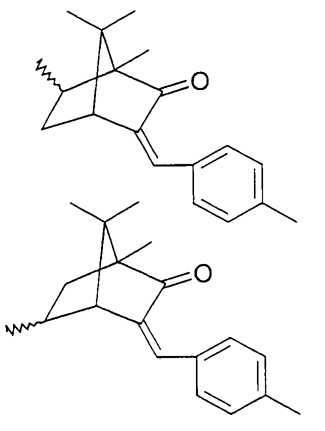 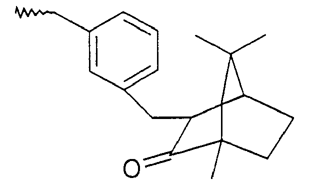 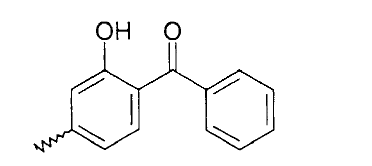 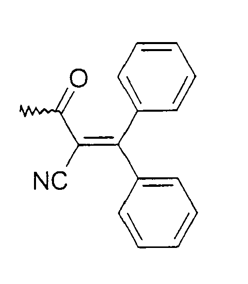 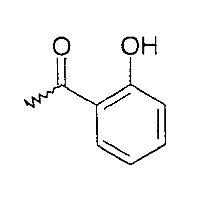 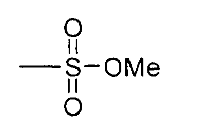 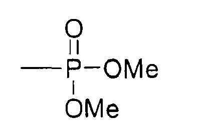 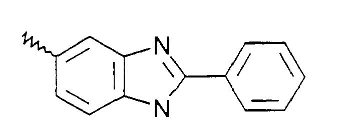 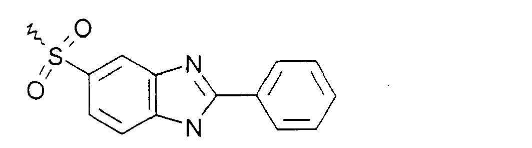 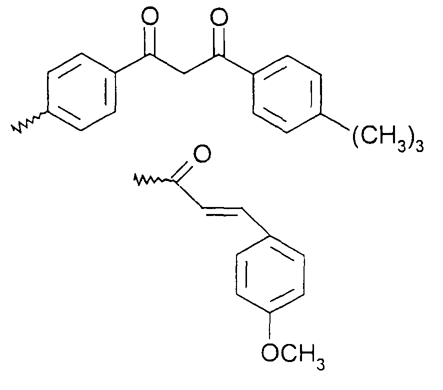 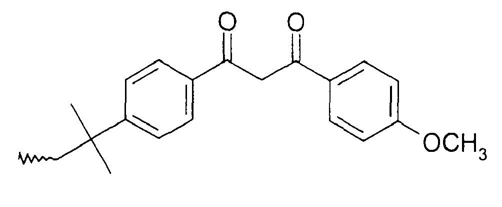
wobei Me steht für ein Proton oder ein Alkalimetallion, insbesondere ein Natrium- oder Kaliumion.

Die Reste können also als Ether oder als Ester an den Grundkörper gebunden sein. Derartige Verbindungen werden in der Deutschen Patentanmeldung DE 10003785.2 beschrieben. Zusammensetzungen, die derartige Coumaranonderivate enthalten, wirkenb besonders hautschonend, die diese Verbindungen antioxidante und radikalfangende Wirkung zeigen.

Sehr gute Eigenschaften besitzt der Grundkörper 4,6,3',4'-Tetrahydroxybenzylcoumaranon-3. Dies entspricht der oben stehenden Formel, wobei X = -CH₂- und R¹ = R² = R³ = R⁴ = H ist. Die Löslichkeit dieser Verbindung in Wasser kann verbessert werden, indem beispielsweise die Reste R¹, R², R³ und R⁴ als Sulfat- oder Phosphatgruppen gewählt werden. Besonders geeignet ist ein Gemisch aus Mono-, Di- und Trisulfat, das im weiteren als "sulfatisiertes Coumaranon" bezeichnet wird. Besonders hervorgehoben ist das Trisulfat (X = -CH₂-; R¹ = R³ = R⁴ = SO₃Me, R² = H), das durch die folgende Formel dargestellt wird.

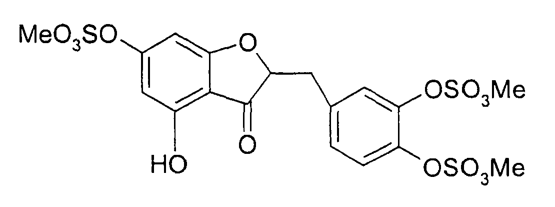

Alle Verbindungen oder Komponenten, die in den Zubereitungen verwendet werden können, sind entweder bekannt und käuflich erwerbbar oder können nach bekannten Verfahren synthetisiert werden.

Die Lichtschutzmittel enthaltenden kosmetischen und pharmazeutischen Zubereitungen sind in der Regel auf der Basis eines Vehikels, welches mindestens eine Ölphase enthält. Es sind aber auch Zubereitungen allein auf wässriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können somit in flüssiger, pastöser oder fester Form vorliegen, beispielsweise als Wasser-in-Öl-Cremes, Öl-in-Wasser-Cremes und -Lotionen, W/O/W-Systeme oder O/W/O-Systeme, Aerosol-Schaumcremes, Gele, Öle, Fettstifte, Puder, Sprays oder alkoholisch-wässrige Lotionen. Ferner können sie auch als mikronisierte Systeme oder als PIT-(Phasen-Inversion-Temperatur)-Emulsionen formuliert sein.

Übliche Ölkomponenten in der Kosmetik sind beispielsweise Paraffinöl, Glycerylstearat, lsopropylmyristat, Düsorpopyladipat, 2-Ethylhexansäureacetylstearylester, hydriertes Polyisobuten, Vaseline, Caprylsäure/Caprinsäure-Triglyceride, mikrokristallines Wachs, Lanolin, Mineralöle, Mineralwachse, Ester von Fettsäuren mit Akoholen niedriger C-Zahl, z.B. mit Isopropanol, Propylenglykol oder Glycerin, Alkylbenzoate, Silikonöle wie Dimethylpolysiloxane, Diethylpolysiloxane, Diphenylpolysiloxane und Stearinsäure.

Die Zubereitungen können kosmetische AdjUV-Anzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Fette und Wachse, Lanolin, Treibmittel, Stabilisatoren, Antioxidantien, Bakterizide, Farbstoffe und/oder Pigmente, welche das Mittel selbst oder die Haut färben, und andere in der Kosmetik gewöhnlich verwendete Ingredienzien.

Als Emulgatoren kommen vorzugsweise bekannte W/O- und daneben auch O/W-Emulgatoren wie etwas Polyglycerinester, Sorbitanester oder teilveresterte Gylceride in Betracht. Typische Beispiele für Fette sind Glyceride; als Wachse sind u.a. Bienenwachs, Carnaubawachs, Paraffinwachs oder Mikrowachse, gegebenenfalls in Kombination mit hydrophilen Wachsen zu nennen. Als Stabilisatoren können Metallsalze von Fettsäuren wie z.B: Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Ferner können auch Pflanzenextrakte, Eiweißhydrolysate und Vitaminkomplexe zugesetzt werden.

Als Antioxidantien können beispielsweise Aminosäuren, Imidazole, Peptide, Carotinoide, α-Hydroxysäuren, ungesättigte Fettsäuren, Vitamin A, C und/oder E und geeignete Derivate dieser genannten Stoffe verwendet werden.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B.
Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordohydroguajaretsäure, Trihydroxybutyrophenon, Quercitin, Harnsäure und deren Derivate. Mannose und deren Derivate, Zink und dessen Derivate (z.B. ZnO, ZnSO4), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherot, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004).

In erfindungsgemäßen Formulierungen liegen die Chinoxalinderivate der Formeln Ik und II oder zu den Antioxidantien dabei üblicherweise in Gewichtsverhältnissen von 10.000:1 bis 1:5, vorzugsweise von 500:1 bis 1:2 und insbesondere bevorzugt von 50:1 bis 1:1 vor.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die wässrige Phase der erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol Ethylenglykolmonoethyl- oder -monobutylether oder analoge Produkte, ferner Alkohole wie Ethanol, Isopropanol, 1,2-Propandiol sowie insbesondere ein oder mehrere Verdickungsmittel, wie z.B. Siliciumdioxid, Aluminiumsilikate, Polysaccharide bzw. deren Derivate, z.B. Hyaluronsäure, Xanthangummi, Hydroxypropylemethylcellulose, oder ein Polyacrylat aus der Gruppe der sogenannten Carbopole.

Gebräuchliche Filmbildner sind beispielsweise Hydrocolloide wie Chitosan, mikrokristallines Chitosan oder quaternäres Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösungen, p-Hydroxybenzoat oder Sorbinsäure. Als Perlglanzmittel kommen beispielsweise Glycoldistearinsäureester wie Ethylenglydoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentrationen von 0,001 bis 0,1 Gew.-%, bezogen auf die gesamte Mischung, eingesetzt.

Gegebenenfalls können die erfindungsgemäßen Sonnenschutzmittel auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Zubereitungen geeignet sind, eingesetzt werden. Solche können sowohl pflanzliche Extrakte als auch synthetische Selbstbräuner, wie z.B. Dihydroxyaceton oder α-Ketole, sein.

Soll das erfindungsgemäße Mittel natürliche oder sensibilisierte Haare vor Sonneneinstrahlung schützen, so kann es als Shampoo, Lotion, Gel oder Emulsion zum Ausspülen vorliegen, wobei die jeweilige Zubereitung vor oder nach dem Schampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Haarspray, Aerosol-Schaumcreme, Dauerwellmittel, Färbe- oder Entfärbemittel der Haare vor. Dieses Mittel kann außer den erfindungsgemäßen Lichtschutzfiltern (VIS- und/oder IR-Filter) oder der erfindungsgemäßen Kombination von Lichtschutzfiltern verschiedene, in diesem Mitteltyp verwendete AdjUV-Anzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Die erfindungsgemäßen Lichtschutzfilter lassen sich direkt ohne weitere vorbereitende Maßnahmen in kosmetischen Zubereitungen einarbeiten.

Diese Substanzen bieten ferner den großen Vorteil, keine toxischen oder allergischen Reaktionen gegenüber der Haut zu zeigen.

Vorteilhaft können die Lichtschutz Zubereitungen erfindungsgemäß, wie vorstehend schon beschrieben, weitere UV-Filtersubstanzen enthalten, wobei die Gesamtmenge der Filtersubstanzen z.B. 0,1 Gew.-% bis 30 Gew.-%, vorzugsweise 0,5 bis 10 Gew.-%, insesondere 1 bis 6 Gew.-% beträgt, bezogen auf das Gesamtgewicht der Zubereitungen.

Weiterhin können die erfindungsgemäßen Zubereitungen auch als pharmazeutische Mittel zur vorbeugenden Behandlung von Entzündungen und Allergien der Haut sowie auch in bestimmten Fällen zur Verhütung bestimmter Krebsarten verwendet werden. Das erfindungsgemäße pharmazeutische Mittel kann oral oder topisch verabreicht werden. Für die orale Verabreichung liegt die pharmazeutische Zubereitung in Form von u.a. Pastillen, Gelatinekapseln, Dragees, als Sirup, Lösung, Emulsion oder Suspension vor. Die topische Anwendung erfolgt beispielsweise als Salbe, Creme, Gel, Spray, Lösung oder Lotion.

Die erfindungsgemäßen kosmetischen und pharmazeutischen Zubereitungen können mit Hilfe von Techniken hergestellt werden, die dem Fachmann wohl bekannt sind.

Ebenso positiv sind die Eigenschaften von Verbindungen der Formeln Ik und/oder II zu werten für eine Verwendung in Nahrungsmitteln, als Nahrungsergänzungsmittel,als diätetisches Mittel oder als "functional food". Die weiteren zu Nahrungsmitteln ausgefahrten Erläuterungen gelten sinngemäß auch für Nahrungsergänzungsmittel und für "functional food".

Die Nahrungsmittel, die mit einer oder mehreren der erfindungsgemäßen Verbindungen angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Nahrungsmittel, die mit einer oder mehreren Verbindungen der Formeln Ik und/oder II angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkampott, Aprikosennektar, Tomatensaft, Tomatensoße, Tomatenpüree usw. Weitere Beispiele für Nahrungsmittel, die mit einer oder mehreren Verbindungen der Formel Ik und oder II angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren der erfindungsgemäßen Verbindungen angereichert zu werden, beispielsweise Multivitaminpräparate, Mineralstoffrnischungen oder gezuckerter Saft. Als weitere Beispiele für Nahrungsmittel seien Nahrungsmittelzubereitungen, beispielsweise zubereitete Cerealien, Gebäck. Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die mit einer oder mehreren Verbindungen der Formeln Ik und/oder II angereichert werden können, umfassen somit alle genießbaren Kombinationen von Kohlehydraten, Lipiden, Proteinen, anorganischen Elementen, Spurenelementen, Vitaminen, Wasser oder aktiven Metabolite von Pflanzen und Tieren.

Die Nahrungsmittel, die mit einer oder mehreren Verbindungen der Formeln Ik und/oder II angereichert werden können, werden vorzugsweise oral angewendet, z.B. in Form von Speisen, Pillen, Tabletten, Kapseln, Pulver, Sirup, Lösungen oder Suspensionen.

Diese angereicherten Nahrungsmittel können mit Hilfe von Techniken hergestellt werden, die dem Fachmann wohl bekannt sind.

Auch ohne weitere Ausführungen wird davon ausgegangen, dass ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die nachfolgenden Beispiele sollen die vorliegende Erfindung verdeutlichen, ohne sie einzuschränken. Alle Mengenangaben, Anteile und Prozentanteil sind, soweit nicht anders angegeben, auf das Gewicht und die Gesamtmenge bzw. das Gesamtgewicht der Zubereitungen bezogen.

Die vollständige Offenbarung aller vor- und nachstehend aufgeführten Anmeldungen und Veröffentlichungen sind durch Bezugnahme in diese Anmeldung eingeführt.

### Herstellungsbeispiele

### Beispiel A

Darstellung von N-(2-Chinoxalinyl)-4-amino-benzoesäure-isopropylester

1 mmol 2-Chlorchinoxalin und 1,5 mmol 4-Aminobenzoesäure-isopropylester werden in 5 ml *n*-Propanol gelöst und 4 h bei einer Temperatur von 120 °C auf Rückfluß erhitzt. Der während der Reaktion ausgefallene Feststoff wird nach dem Abkühlen durch Filtration abgetrennt und mit Aktivkohle aus Essigester umkristallisiert. Es werden 91 % der Verbindung N-(2-Chinoxalinyl)-4-amino-benzoesäure-isopropylester als gelbliches Pulver erhalten.
**Charakterisierung:**

| **IR (KBr):** | | |
|---|---|---|
| Wellenzahl [cm-1] = | 3333 | (ν(N-H), NH) |
| | 3155 | (v(C-H), Ph) |
| | 1720 | (ν(C=O), CO₂R) |
| | 1613 | (ν(C=C), Ph) |
| | 1551 | (ν(C=C), Ph) |
| | 1290 | ((Gerüst), Ph+CO) |
| | 760 | (γ(C-H), Ph) |
| | 699 | (γ(C-H), Ph) |
| **MS:** | | |
| *m*/*z (*%*)* = | 309 (5) [M⁺+2], 308 (35) [M⁺+1], 307 (91) [M⁺], 264 100), 248 (43), 220 (41), 192 (6), 166(3), 145(2), 129(16), 118(31), 118 (31), 102(23), 91 (3) | |
| **¹H-NMR (300 MHz, d₆-DMSO):** | | |
| δ = | 8.40 | (s, 1 H, C-3) |
| | 8.03 | (m, 1 H, Aryl, arom.) |
| | 7.99 | (m, 1 H, Aryl, arom.) |
| | 7.80- 7.89 | (m, 4H, Hetaryl, arom.) |
| | 7.58 | (m, 1 H, Aryl arom.) |
| | 7.48 | (m, 1 H, Aryl arom.) |
| | 5.18 | (sep, 1 H, CH) |
| | 1.32 | (s, 3H, CH₃) |
| | 1.24 | (s, 3H, CH₃) |

### Beispiel B

### Darstellung von N-(2-Chinoxalinyl)-2-amino-pyrimidin

Man löst 1 mmol 2-Chlorchinoxalin und 1, 5 mmol 2-Amino-pyrimidin in einem inerten Lösungsmittel und erhitzt 4 h unter Rückfluß. Der während der Reaktion ausgefallene Feststoff wird nach dem Abkühlen durch Filtration abgetrennt und durch Umkristallisation gereinigt. Man erhält so N-(2-Chinoxalinyl)-2-amino-pyrimidin.
**Charakterisierung:**

| | | |
|---|---|---|
| **IR (KBr):** | | |
| Wellenzahl [cm-1] = | 3427 | (v(N-H), NH) |
| | 3021 | (ν(C - H), Ph) |
| | 1597 | (ν(C = C), Ph) |
| | 1493 | (ν(C = C), Ph) |
| | 1262 | ((Gerüst), Ph) |
| | 814 | (γ (C-H), Ph) |
| | 788 | (γ(C-H), Ph) |
| **MS:** | | |
| *m*/*z (*%*)* = | 224 (97) [M⁺+1], 223 (100) [M⁺], 222 (12) [M⁺-1], 195 (6), 170 (7), 143(4) 129 (9), 102 (16), 90 (11), 79 (18) | |
| **¹H-NMR (300 MHz, d₆-DMSO):** | | |
| δ = | 10.71 | (br. s, 1H, NH) |
| | 9.71 | (s, 1 H, Hetaryl, arom.) |
| | 8.64 | (d, 2H, Hetaryl, arom.) |
| | 7.66-8.03 | (m, 4H, Hetaryl, arom.) |

### Beispiel C

### Herstellungsbeispiele

Analog Beispiel B erhält man aus den entsprechenden Ausgangsprodukten folgende Chinoxalinderivate:
a) aus 2-Chlor-2-phenyl-chinoxalin und 4-Aminobenzoesäure die N-[2-(3-Phenyl)-chinoxalinyl]-4-amino-benzoesäure;
Charakterisierung

| | | |
|---|---|---|
| **IR (KBr):** | | |
| Wellenzahl | 3421 | (v(N-H), NH) |
| [com-1]= | | |
| | 3085 | (v(C-H), Ph) |
| | 1709 | (ν(C=O)) |
| | 1618 | (ν(C=C), Ph) |
| | 1526 | (ν(C=C), Ph) |
| | 1256 | ((Gerüst), Ph) |
| | 814 | (γ(C-H), Ph) |
| | 742 | (γ(C-H), Ph) |
| **MS:** | | |
| *m*/*z (*%*)* = | 342 (16) [M⁺+2], 341 (75) [M⁺1], 340 (100) [M⁺], 296 (18), 264 (2), 220 (11), 194 (17), 166 (5), 137 (13),102 (17), 90 (23), 77 (21) | |
| **¹H-NMR (300 MHz, d₆-DMSO):** | | |
| δ = | 7.80-7.94 | (m, 4H, Hetaryl, arom.) |
| | 7.51-7.65 | (m, 5H, Aryl, arom.) |
| | 7.43 | (br. s, 1H, OH) |
| | 7.23 | (m, 4H, Aryl, arom.) |

b) aus 2-Chlorchinoxalin und 2,4,6-Trifluoroanilin das N-[Bis-(2-Chinoxalinyl)]-2,4,6-trifluoro-anilin;
**Charakterisierung:**

| | | |
|---|---|---|
| **IR (KBr):** | | |
| Wellenzahl [cm-¹] = | 2925 | (ν(C_{sp²} -H), Ph), |
| | 1535 | (ν (C=C), Ph), |
| | 1122 | (ν(C-F)), |
| | 758 | (γ (C-H), Ph). |
| **¹H-NMR (300 MHz, d₆-DMSO):** | | |
| δ = | 11.48 | (s, 1 H), |
| | 10.32 | (s, 1 H, arom.), |
| | 8.13-8.20 | (m, 1 H, arom.), |
| | 8.07-8.13 | (m, 1 H, arom.), |
| | 8.00-8.13 | (dd, 1 H, arom., ³J = 8.1 Hz, ⁴*J* = 0.8 Hz), |
| | 7.76-7.83 | (m, 2 H, arom.), |
| | 7.64 | (dd, 1 H, 8-H, *J* = 8.3 Hz, ⁴*J* = 1.4 Hz), |
| | 7.59 | (dt, 1 H, 7-H, ³*J* = 8.4 Hz, ⁴*J* = 1.4 Hz), |
| | 7.46 | (dt, 1 H, 6-H, ³*J* = 6.6 Hz, ⁴*J* = 1.7 Hz), |
| | 7.19 | (s, 3 H, arom.), |
| | 6.75-6.85 | (m, 2 H, arom.). |
| **MS:** | | |
| *m*/*z* (%) = | 405 (2) [M⁺ +2], 404 (16) [M⁺ +1], 403 (64) [M⁺], 384 (100), 357 (5), 281 (6), 256 (6), 202 (12), 144 (27), 129 (12), 102 (17), 90 (13), 77 (11), 57 (6). | |

c) aus 2-Chlorchinoxalin und 2-Amino-4,5-dimethoxy-benzoesäure die N-(2-Chinoxalinyl)-2-amino-4,5-dimethoxy-benzoesäure;
Charakterisierung:
Schmelzpunkt :248°C.

| | | |
|---|---|---|
| **IR (KBr):** | | |
| Wellenzahl [cm-¹] = | 3115 | (ν(N-H), NH), |
| | 2991 | (ν(C_{sp²}-H), Ph), |
| | 1743 | (ν(C=O), CO₂H), |
| | 1637 | (ν(C=C), Ph), |
| | 1533 | (ν(C=C), Ph), |
| | 1246 | (δ(C-O-H), CO₂H), |
| | 753 | (γ (C-H), Ph). |
| **¹H-NMR (300 MHz, d₆-DMSO):** | | |
| δ = | 11.42 | (s, 1 H, NH), |
| | 9.06 | (s, 1 H, 6'-H), |
| | 8.52 | (s, 1 H, 3-H), |
| | 7.88 | (dd, 1 H, 5-H, ³*J* = 8.1 Hz, ⁴*J* = 0.9 Hz), |
| | 7.76 | (dd, 1 H, 8-H, ³*J* = 8.2 Hz, ⁴*J* = 1.0 Hz), |
| | 7.67 | (dt, 1 H, 7-H, ³*J* = 7.6 Hz, ⁴*J* = 1.3 Hz), |
| | 7.51 | (dt, 1 H, 6-H, ³*J* = 7.5 Hz, ⁴*J* = 1.4 Hz), |
| | 7.46 | (s, 1 H, 3'-H), |
| | 3.95 | (s, 3 H, OCH₃), |
| | 3.77 | (s, 3 H, OCH₃). |
| **¹³C-NMR (75.4 MHz, d₆-DMSO):** | | |
| δ = | 169.62 | (CO₂H), |
| | 153.31 | (C-4'), |
| | 148.41 | (C-2), |
| | 142.38 | (C-5'), |
| | 141.13 | (C-3), |
| | 139.83 | (C-8a), |
| | 138.53 | (C-2'), |
| | 137.04 | (C-4a), |
| | 130.08 | (C-7), |
| | 128.34 | (C-5), |
| | 126.76 | (C-6), |
| | 125.55 | (C-8), |
| | 112.98 | (C-6'), |
| | 106.28 | (C-1'), |
| | 102.66 | (C-3'), |
| | 55.51 | (OCH₃). |
| **MS:** | | |
| *mlz* (%) = | 327 (2) [M⁺ +2], 326 (19) [M⁺ +1], 325 (100) [M⁺], 307, (30), 292 (25), 280 (83), 266 (45), 236 (13), 221 (5), 206 (10), 193 (27), 179 (3), 145 (3), 129 (50), 102 (36), 90 (7), 76 (8), 64 (4). | |

d) aus 2-Chlorchinoxalin und 4-Aminomethyl-benzoesäure die N-(2-Chinoxalinyl)-4-aminomethyl-benzoesäure;
e) aus 2-Chlorchinoxalin und 3,4,5-Trimethoxy-anilin das N-(2-Chinoxalinyl)-3,4,5-trimethoxy-anilin;
f) aus 2-Chlorchinoxalin und 2-Amino-anisol das N-(2-Chinoxalinyl)-2-amino-anisol;
**Charakterisierung:**
Schmelzpunkt : 216°C.

| | | | |
|---|---|---|---|
| **IR (KBr):** | | | |
| Wellenzahl [cm-¹] = | | 3047 | (v (C_{sp²}-H), Ph), |
| | | 2784 | (v (N-H), NH), |
| | | 1656 | (v (C=C), Ph), |
| | | 1509 | (v (C=C), Ph), |
| | | 1251 | (ν(C=O), R-O-R), |
| | | 835 | (γ(C-H), Ph), |
| | | 756 | (γ(C-H), Ph). |
| **¹H-NMR (300 MHz, d₆-DMSO):** | | | |
| δ = | 10.07 | (s, 1 H, NH), | |
| | 8.56 | (s, 1 H, 3-H), | |
| | 7.88 | (d, 2 H, 2'-H, 6'-H, ³*J* = 9.0 Hz), | |
| | 7.84 | (dd, 1 H, 5-H, ³*J* = 8.4 Hz, ⁴*J* = 0.7 Hz), | |
| | 7.68 | (dd, 1 H, 8-H, ³*J* = 8.3 Hz, ⁴*J* = 1.4 Hz), | |
| | 7.63 | (dt, 1 H, 7-H, ³J = 8.2 Hz, ⁴J = 1.4 Hz), | |
| | 7.44 | (dt, 1 H, 6-H, ³J = 7.4 Hz, ⁴*J* = 1.6 Hz), | |
| | 6.99 | (d, 2 H, 3'-H, 5'-H, ³J = 9.0 Hz), | |
| | 3.77 | (s, 3 H, OCH₃). | |
| **¹³C NMR (75.4 MHz, d₆-DMSO):** | | | |
| δ = | 154.71 | (C-4'), | |
| | 149.42 | (C-2), | |
| | 140.75 | (C-3), | |
| | 140.15 | (C-8a), | |
| | 136.47 | (C-4a), | |
| | 133.09 | (C-1'), | |
| | 129.99 | (C-7), | |
| | 128.36 | (C-5), | |
| | 125.66 | (C-6), | |
| | 124.52 | (C-8), | |
| | 120.45 | (C-2', C-6'), | |
| | 114.03 | (C-3', C-5'), | |
| | 55.15 | (OCH₃). | |
| **MS:** | | | |
| *m*/*z* (%) = | 253 (2) [M⁺ +2], 252 (21) [M⁺ +1], 251 (100) [M⁺], 236 (87), 220 (2), 208 (19), 181 (4), 154 (1), 129 (44), 118 (8), 102 (39), 90 (12), 76 (15), 63 (14). | | |

g) aus 2-Chlorchinoxalin und 2-Amino-benzophenon das N-(2-Chinoxalinyl)-2-amino-benzophenon.
**Charakterisierung:**
Schmelzpunkt: 158°C.

| | | |
|---|---|---|
| **IR (KBr):** | | |
| Wellenzahl [cm⁻¹] = | 3274 | (v (N-H), NH), |
| | 3123 | (ν(C_{sp²} -H), Ph), |
| | 1603 | (v (C=C), Ph), |
| | 1534 | (v (C=C), Ph), |
| | 1265 | (Gerüst Ph-CO-), |
| | 755 | (γ (C-H), Ph), |
| | 698 | (γ (C-H), Ph). |
| **¹H-NMR (300 MHz, d₆-DMSO):** | | |
| δ = | 10.16 | (s, 1 H, NH), |
| | 8.40 | (s, 1 H, 3-H), |
| | 7.88 | (d, 1 H, 5-H, ³*J* = 7.9 Hz), |
| | 7.76 | (dd, 1 H, 8-H, ³*J* = 7.9 Hz, ⁴*J* = 1.6 Hz), |
| | 7.73 | (dd, 1 H, arom., ³*J* = 8.4 Hz, ⁴*J* = 1.4 Hz), |
| | 7.58-7.70 | (m,2 H, arom.), |
| | 7.36-7.50 | (m, 5 H, arom.), |
| | 7.27 | (dt, 1 H, arom., ³*J* = 7.6 Hz, ⁴*J* = 1.3 Hz), |
| | 7.06 | (dd, 1 H, arom., ³*J* = 8.1 Hz, ⁴*J* = 1.4 Hz). |
| **¹³C-NMR (75.4 MHz, d₆-DMSO):** | | |
| δ = | 157.65 | (C-1'), |
| | 148.98 | (C-2), |
| | 139.86 | (C-3), |
| | 137.76 | (C-8a), |
| | 137.13 | (C-4a), |
| | 132.53 | (Cₜₑᵣₜ. arom.), |
| | 132.04 | (C-4"), |
| | 130.40 | (C_{tert.} arom.), |
| | 129.76 | (C-7), |
| | 129.57 | (C-2", C-6"), |
| | 128.30 | (C-5), |
| | 128.10 | (C-3", C-5"), |
| | 125.40 | (C-6), |
| | 124.92 | (C-8), |
| | 122.90 | (C_{tert.} arom.), |
| | 122.63 | (C_{tert.} arom.). |
| MS: | | |
| m/z (%) = | 327 (1) [M+ +2], 326 (10) [M+ +1], 325 (41) [M+], 296 (32), 248 (10), 220 (100), 196 (5), 152 (5), 118 (5), 105 (12), 92 (10), 77 (34), 65 (5). | |

h) aus 2-Chlor-3 Phenyl-chinoxalin und 2-Amino-4,5-dimethoxybenzoesäure die 2-(3-Phenyl-chinoxalin-2-yl-amino)-4,5-dimethoxybenzoesäure
Charakterisierung:
Schmelzpunkt :126°C.

| | | | |
|---|---|---|---|
| **IR (KBr):** | | | |
| Wellenzahl [cm-¹] = | | 3422 | (v (N-H), NH), |
| | | 1995 | (ν(C_{sp²}-H), Ph), |
| | | 2828 | (ν(C_{sp³}-H), CH₃), |
| | | 1604 | (ν(C=O), CO₂H), |
| | | 1537 | (ν(C=C), Ph), |
| | | 1228 | (ν(C-O), R-O-R), |
| | | 863 | (γ(C-H), Ph), |
| | | 753 | (γ(C-H), Ph). |
| **¹H-NMR (300 MHz, d₆-DMSO):** | | | |
| δ = | 8.31 | | (s, 1 H, NH), |
| | 7.83 - 7.90 | | (m, 3H, arom.), |
| | 7.66 | | (dt, 1 H, arom., ³*J* =7.2 Hz, ⁴*J* = 1.0 Hz), |
| | 7.55-7.66 | | (m, 5 H, arom.), |
| | 7.47 | | (dt, 1 H, 6-H, ³*J* = 7.4 Hz, ⁴*J* = 1.5 Hz), |
| | 7.38 | | (dt, 1 H, arom., ³*J* = 8.7 Hz, ⁴*J* = 2.9 Hz), |
| | 6.93 | | (d, 1H, arom., ³*J* = 8.8 Hz), |
| | 3.77 | | (s, 3 H, OCH₃), |
| | 3.76 | | (s, 3 H, OCH₃). |
| **¹³C-NMR (75.4 MHz, d₆-DMSO):** | | | |
| δ = | 148.33 | | (C-2) |
| | 147_{.}66, 147.20, 144.53 | | (C_{quart.} arom.), |
| | 140.01 | | (C-1 "), |
| | 136.98 | | (C-8a), |
| | 136.65 | | (C-4a), |
| | 133.36 | | (C-2'), |
| | 128.77 | | (C-7), |
| | 129,52 | | (C-3", C-5"), |
| | 128.72 | | (C-2", C-4", C-6"), |
| | 128.27 | | (C-5), |
| | 125.91 | | (C-6), |
| | 125.04 | | (C-8), |
| | 112.93.111.9,106.3 | | (C_{quart} arom.), |
| | 55.77 | | (OCH₃), |
| | 55.35 | | (OCH₃). |
| **MS (EI):** | | | |
| *m*/*z* (%) = | 391 [M⁺] (2), 359 (39), 357 (100), 342 (61), 327 (1), 299 (4),270 (6), 220 (2),205 (25), 179 (10),150 (3),102 (10), 90 (4), 77 (17), 65 (2). | | |
| **MS (FD):** | | | |
| *m*/*z* (%) = | 401 [M⁺] (2), 391 (6), 357 (100), 178 (2), 78 (4). | | |

### Beispiele E

### Darstellung von N,N-Benzoyl-(2-Chinoxalinyl)-4-amino-benzoesäure

Zu einer Suspension von 0,55 mmol AgOTf (Silbertriflat) und 0,275 mmol TiCl₄ (Titantetrachlorid) in 5 ml Dichlormethan wird unter Stickstoffatmosphäre portionsweise eine Mischung von 2 mmol Benzoesäureanhydrid und 2 mmol N-(2-Chinoxalinyl)-4-amino-benzoesäuretrimethylsilylester [herstellbar durch Umsetzung von N-(2-Chinoxalinyl)-4-amino-benzoesäure mit Trimethylsilylcyanid in Acetonitril unter Stickstoffatmosphäre] in 7 ml Dichlormethan gegeben. Anschließend wird eine Lösung von 2 mmol 2-Propyl-trimethylsilylether (herstellbar durch Umsetzung von Trimethylchlorsilan mit N,N-Dimethylanilin in einer Lösung von Propan-2-ol) zugetropft und die Reaktionsmischung 20 h bei Raumtemperatur gerührt. Nach Zugabe von wässriger gesättigter NaHCO₃- Lösung wird mit Chloroform extrahiert und die organische Phase nach dem Abtrennen über Natriumsulfat getrocknet. Die weitere Reinigung erfolgt säulenchromatographisch an Kieselgel mit Cyclohexan / Essigester (1:1). N,N-Benzoyl-(2-Chinoxalinyl)-4-amino-benzoesäure kann als blassgelbes Pulver in einer Ausbeute von 27 % erhalten werden.
**Charakterisierung:**

| | | | |
|---|---|---|---|
| **IR (KBr):** | | | |
| Wellenzahl [cm-1] = | | 3354 | (ν(-NR₂) |
| | | 3123 | (ν(C-H), Ph) |
| | | 1789 | (ν(O=C-NR₂) |
| | | 1712 | (ν(C=O)) |
| | | 1600 | (ν(C=C), Ph) |
| | | 1521 | (ν(C=C), Ph) |
| | | 1228 | (ν(Gerüst), Ph-C-O-) |
| | | 758 | (γ(C-H), Ph) |
| | | 708 | (γ(C-H), Ph) |
| **MS:** | | | |
| *m*/*z (*%*)* = | | 371 (10) [M⁺+2], 370 (32) [M⁺+1], 369 (100) [M⁺], 345 (8), 264(6), 220 (7), 122 (8), 105 (4), 78 (9) | |
| **¹H-NMR (300 MHz, d₆-DMSO):** | | | |
| δ = | 8.64 | | (s, 1H, 3-H) |
| | 7.49-7.68 | | (m, 5H, Benzoyl, |
| | | | arom.) |
| | 7.82-7.97 | | (m, 4H, Aryl, arom.) |
| 8.09-8.26 | | (m, 4H, Hetaryl, aromat.) | |

### Beispiel F

### Darstellung von N-(2-Chinoxalinyl)J-4-amino-benzoyl-glutaminsäurediethylester

Eine Mischung von 1,2 mmol N-(2-Chinoxalinyl)-4-amino-benzoesäure und 1,3 mmol Diethyl-L-glutamat-hydrochlorid in 24 ml trockenem DMF werden bei Raumtemperatur und unter Stickstoffatmosphäre zu einer Lösung von 1,3 mmol Diethyl-cyano-phosphonat in 2,5 mmol Triethylamin gegeben. Nach 1,5 h Rühren wird die Lösung in einem Gemisch von Essigester/ Benzol (Verhältnis 3:1) aufgenommen und mit Wasser und wässriger gesättigter NaHCO₃-Lösung gewaschen. Nach Abtrennen der organischen Phase wird diese über Natriumsulfat getrocknet und durch Säulenchromatographie an Kieselgel mit Cyclohexan/ Essigester (1:2,5) gereinigt. Man erhält N-(2-Chinoxalinyl)J-4-amino-benzoyl-glutaminsäurediethylester in einer Ausbeute von 71 %.
Charakterisierung:

| | | |
|---|---|---|
| **IR (KBr):** | | |
| Wellenzahl [cm-1] = | 3383 | (v(N-H), NH) |
| | 3312 | (ν(N-H), NH) |
| | 2982 | (v(C-H), Alkyl) |
| | 1747 | (ν(C=O) |
| | 1642 | (ν(C=C), Ph) |
| | 1544 | (ν(C=C), Ph) |
| | 1270 | ((Gerüst), Ph) |
| | 856 | (γ(C-H), Ph) |
| | 765 | (γ(C-H), Ph) |
| **MS:** | | |
| m/z% = *m*/*z (*%*)* = | 451 (4) [M⁺+1], 450 (14) [M⁺], 404 (9),377 (2), 322 (3), 293 (15), 264 (12), 248 (100), 220 (26), 202 (10),151 (3), 120 (42), 84 (35) | |
| **¹H-NMR (300 MHz, d₆-DMSO):** | | |
| δ = | 8.48 | (s, 1 H, H-3) |
| | 7.84-7.96 | (m, 4H, Hetaryl, arom.) |
| | 7.03-7.69 | (m, 2H, Aryl, arom.) |
| | 7.48-7.54 | (m, 2H, Aryl, arom.) |
| | 4.80 | (überiagert + 1 H, CH) |
| | 4,24 | (dt, 2H, CH₂) |
| | 4.12 | (dt, 2H, CH₂) |
| | 2.46 | (q, 2H, CH₂ CH₃) |
| | 2.16 | (q, 2H, CH₂ CH₃) |
| | 1.28 | (t, 3H, CH₃) |
| | 9.20 | (t, 3H, CH₉) |

### Beispiel I: N - 2-[3-(Ethoxycarbonyl ) - chinoxalinyl]-4-aminobenzoesäure

Herstellung: 2-Chlor-3-Ethoxycarbonylester-chinoxalin(100g, 4.46 mmol) und p-Aminopbenzoesäure (0.91 g, 6,69 mmol) werden in 20 mL EtOH unter Rückfluß erhitzt. Das Produnkt fällt währens der Reaktion als gelber Feststoff aus. Nach dem Absaugen erfolgt die Umkristallisation aus EtOH. Man erhält die N-2[3-(Ethoxycarbonyl)-Chinoxalinyl]-4-amino-benzoesäure als gelben Feststoff.
Charakterisierung:

| | | | |
|---|---|---|---|
| **IR (KBr):** | | | |
| Wellenzahl [cm-1] = | | 3480 | (v(O-H), OH) |
| | | 3270 | (v(N-H), NH) |
| | | 3012 | (v(C - H), Ph) |
| | | 2976 | (v(C - H), Ethyl) |
| | | 1703 | (ν(C = O)CO₂Et) |
| | | 1696 | (ν(C = O)CO₂H) |
| | | 1613 | (v(C = C), Ph) |
| | | 1538 | (v(C = C), Ph) |
| | | 1328 | ((Gerüst), Ph) |
| | | 1292 | ((Gerüst), Ph) |
| | | 768 | (γ(C-H), Ph) |
| **MS:** | | | |
| *m*/*z (*%*)* = | | 338 (19) [M⁺+1], 337 (100) [M⁺], 336 (55), 292 (5), 264 (17), 246 (6) 219 (76), 102 (4), 90 (15), 77 (3) | |
| **¹H-NMR (300 MHz, d₆-DMSO):** | | | |
| δ = | 12.72 | | (br. s, 1 H, OH) |
| | 10.40 | | (br. s, 1H, NH) |
| | 7.91 - 8.25 | | (m, 5H, arom.) |
| | 7.81 - 7.85 | | (m, 2H, Hetaryl, arom.) |
| | 7.57 - 7.66 | | (m, 2H, Hetaryl, arom.) |
| | 4.49 | | (q, 2H, CH₂) |
| | 1.43 | | (t, 3H, CH₃) |

analog wurde hergestellt: N - 2-[3-(Ethoxycarbonyl)-chinoxalinyl] -4 - acetyl-anilin

| | | | |
|---|---|---|---|
| **IR (KBr):** | | | |
| Wellenzahl [cm-1] = | | 3269 | (v(N-H), NH) |
| | | 2987 | (v(C - H), Ethyl) |
| | | 1703 | (ν(C = O)CO₂Et) |
| | | 1676 | (v(C=O)COCH₃) |
| | | 1664 | (v(C = C), Ph) |
| | | 1538 | (v(C = C), Ph) |
| | | 1275 | ((Gerüst), Ph) |
| | | 769 | (γ(C-H), Ph) |
| **MS:** | | | |
| *m*/*z (*%*)* = | | 335 (38) [M⁺], 320 (6), 252 (55), 246 (12), 237 (100), 219 (29), 194 (2), 155 (3), 135 (19), 120 (29), 92 (20), 77 (5) | |
| **¹H-NMR (300 MHz, d₆-DMSO):** | | | |
| δ = | 10.41 | | (br. s, 1H, NH) |
| | 7.99 - 8.11 | | (m, 5H, arom.) |
| | 7.81 - 7.85 | | (m, 2H, Hetaryl, arom.) |
| | 7.57 - 7.66 | | (m, 2H, Hetaryl, arom.) |
| | 4.49 | | (q, 2H, CH₂) |
| | 2.56 | | (s, 3H, COCH₃) |
| | 1.43 | | (t, 3H, CH₃) |

### Beispiel K: 1-(2-Chinoxalinyl) -3 -[4-(iso-propyl)-Phenyl] - prop-2-en-1-on (A) und 1-(2-Chinoxalinyl) -3 -[3,4-(oxolano) -phenyl] - prop-2-en-1-on (B)

In 3ml Ethanol (wasserfrei) wird 1,00 mmol 2-Acetyl-chinoxalin gelöst und bei einer Temperatur von -10 - 0 °C 1,05 mmol des Aldehyds unter Rühren zugegeben. Anschließend wird bei dieser Temperatur 0,40 mmol Base (ethanolisches KOH oder Piperidin) zugetropft. Nach weiterem Rühren der Lösung bei einer Temperatur von 0 °C für 1 h wird langsam auf Raumtemperatur erwärmen und 2 - 6h reagieren lassen. Während der Reaktionszeit scheidet sich ein Feststoff, der nach Filtrieren der Lösung durch Umkristallisation mit Ethanol / Essigester gereinigt werden kann. Die Verbindungen können in Ausbeuten von 46 - 73 % erhalten werden.
Charakterisierung:

| | | |
|---|---|---|
| **IR (KBr):** | | |
| Wellenzahl [cm-1] = | 3063 | (v(C-H), Ph) |
| | 2983 | (v(C - H), Alkyl) |
| | 1670 | (v(C = O)) |
| | 1611 | (ν(C = C), Ph) |
| | 1349 | ((Gerüst), Ph) |
| | 788 | (γ(C-H), Ph) |
| | 769 | (γ(C-H), Ph) |
| MS: | | |
| *m*/*z (*%*)* = | | 301 (100) [M⁺], 286 (10), 272 (43), 258 (27), 243 (3), 230 (55), 203 (1), 172 (17), 155 (4), 130 (84), 114 (5), 103 (7), 91 (2), 77 (2) |
| **¹H-NMR (300 MHz, CDCL₃):** | | |
| δ = | 9.56 | (s, 1H, H-3) |
| | 8.22 | (d, 1H, HC = C) |
| | 8.17 - 8.20 | (m, 1 H, Hetaryl, arom.) |
| | 8.10 - 8.13 | (m, 1 H, Hetaryl, arom.) |
| | 7.95 | (d, 1H, C = CH) |
| | 7.77 - 7.84 | (m, 2H, Hetaryl, arom.) |
| | 7.62 - 7.65 | (m, 2H, arom.) |
| | 7.18 - 7.25 | (m, 2H, arom.) |
| | 2.88 | (sep., 1H, CH) |
| | 1.22 | (s, 3H, CH₃) |
| | 1.19 | (s, 3H, CH₃) |

Charakterisierung B

| | | |
|---|---|---|
| **IR (KBr):** | | |
| Wellenzahl [cm-1] = | 3065 | (v(C-H), Ph) |
| | 2933 | (v(C - H), Alkyl) |
| | 1670 | (v(C = O)) |
| | 1591 | (v(C = C), Ph) |
| | 1268 | ((Gerüst), Ph) |
| | 1243 | ((Gerüst), Ph) |
| | 813 | (γ(C-H), Ph) |
| | 782 | (γ(C-H), Ph) |
| **MS:** | | |
| *m*/*z (*%*)* = | | 304 (100) [M⁺], 290 (4), 288 (2), 276 (9), 258 (37), 232 (12), 230 (47), 204 (15), 72 (6), 159 (3), 130 (71), 102 (17), 91 (11), 77 (2) |
| **¹H-NMR (300 MHz, CDCL₃):** | | |
| δ = | 9.55 | (s,1H, H-3) |
| | 8.17 - 8.24 | (m, 1H, Hetaryl, arom.) |
| | 8.13 | (d, 1H,HC =C) |
| | 8.04 - 8.11 | (m, 1 H, Hetaryl, arom.) |
| | 7.90 | (d, 1H, C = CH) |
| | 7.77 - 7.85 | (m, 2H, Hetaryl, arom.) |
| | 7.24 - 7.26 | (m, 1H, arom.) |
| | 7.15-7.19 | (m, 1H, arom.) |
| | 6.78 - 6.81 | (m, 1H, arom.) |
| | 6.27 | (s, 2H, CH₂) |

### Beispiel P:

In der nachfolgenden Tabelle sind die Strukturformeln erfindungsgemäß einsetzbarer Chinoxalinderivate sowie die Maxima deren UV-A- bzw. UV-B-Absorption angegeben. Die Messung erfolgte in 2-Propanol bei einer Konzentration von 1 mg Substanz pro 100mL Lösungsmittel.

| **Strukturformel** | **UV-A (400 320 nm)** | | **UV-B (280 320 nm)** | |
|---|---|---|---|---|
| | Max. Abs. | λ [nm] | Max. Abs. | λ [nm] |
| 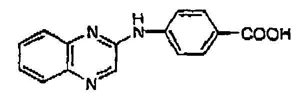 | 0,378 | 374,1 | 1,141 | 297,2 |
| 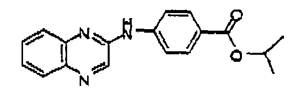 | 0.555 | 374,3 | 1,262 | 306,7 |
| 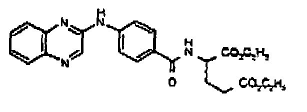 | 0,3076 | 380,0 | 0,756 | 307,0 |
| 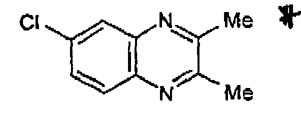 | 0,400 | 322,0 | | |
| 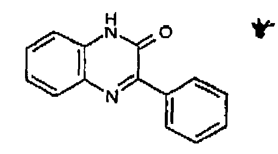 | 0,360 | 365,0 | 0,373 | 305,0 |
| 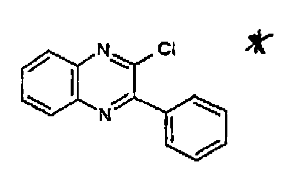 | 0,173 | 335,0 | | |
| 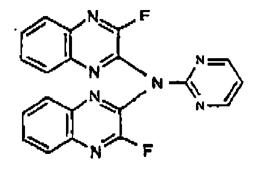 | 0,446 | 329,8 | | |
| 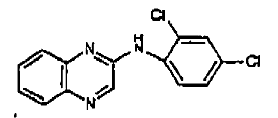 | 0,248 | 365,2 | 0,757 | 280,7 |
| 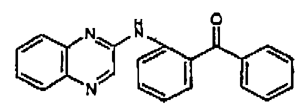 | 0,424 | 395,4 | - | - |
| 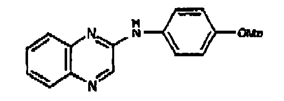 | 0,306 | 386,9- | 0,837 | 291,1 |
| 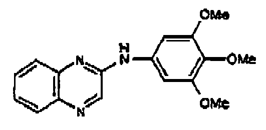 | 0,281 | 386,1 | 0,679 | 275,6 |
| 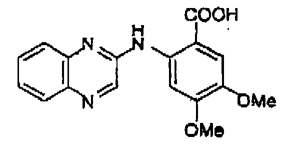 | 0,456 | 380,1 | 0,455 | 300,1 |
| 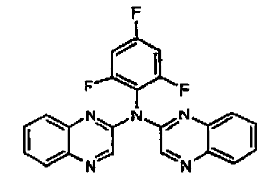 | | | 0,966 | 289,3 |
| 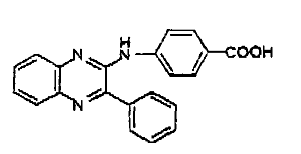 | 0,325 | 376,8 | 0,452 | 303,8 |
| 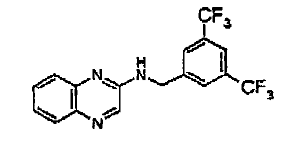 | 0,192 | 359,0 | | |
| 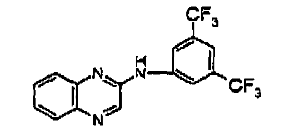 | 0,262 | 359,8 | | |
| 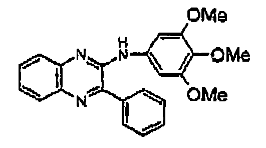 | 0,175 | 390,4 | 0,475 | 267,6 |
| 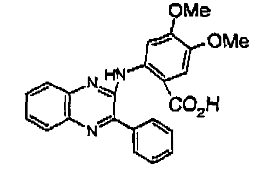 | 0,235 | 393,4 | 0,635 | 285,6 |
| 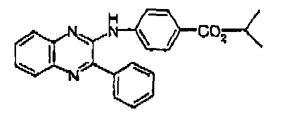 | 0,342 | 381,9 | 0.849 | 305,3 |
| 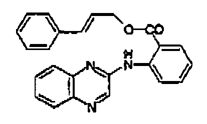 | 0,469 | 386.3 | | |
| 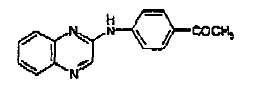 | 0,622 | 374,9 | 1,062 | 314,7 |
| 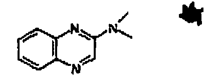 | 0,413 | 378,0 | | |
| 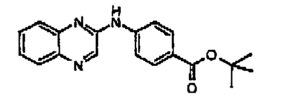 | 0,506 | 374,3 | 1,172 | 306,3 |
| 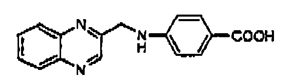 | | | 0,837 | 302,9 |
| 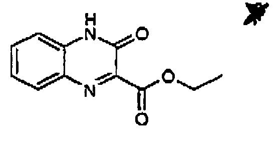 | 0,245 | 355,8 | 0,279 | 290,0 |
| 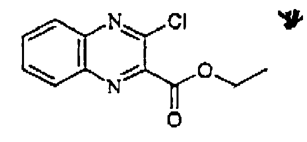 | 0,243 | 322,9 | | |
| 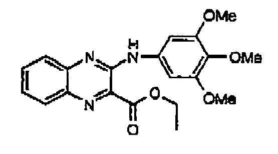 | | | 0,614 | 295,6 |
| 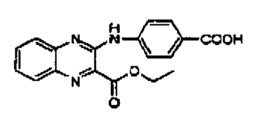 | | | 1,270 | 311,5 |
| 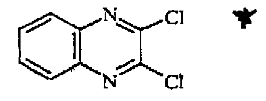 | 0,3379 | 336,5 | | |
| 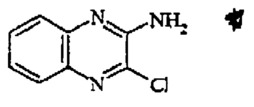 | 0,427 | 353,4 | | |
| 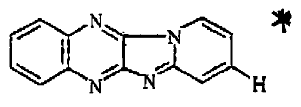 | 0,411 | 348,3 | | |
| 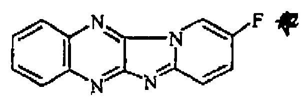 | 0,132 | 344,6 | | |
| 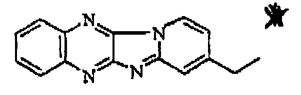 | 0,536 | 356,1 | | |
| 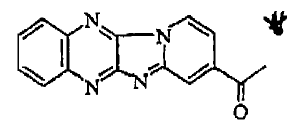 | 0,533 | 364,8 | | |
| 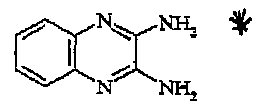 | 0,6269 | 342,5 | | |
| 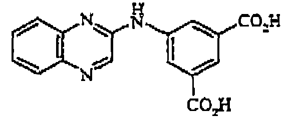 | 0,319 | 372,8 | 0,887 | 286,9 |
| 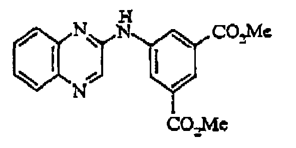 | 0,322 | 368,3 | 0,789 | 287,7 |
| 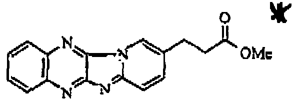 | 0,3747 | 349,5 | | |
| 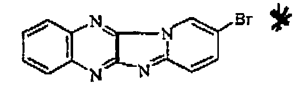 | 0,4113 | 354 | | |
| 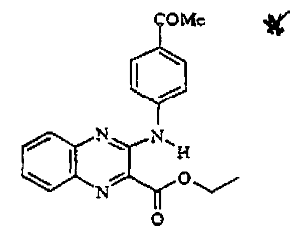 | 1,2265 | 328,5 | | |
| 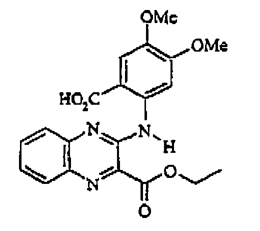 | | | 0,5713 | 295,0 |
| 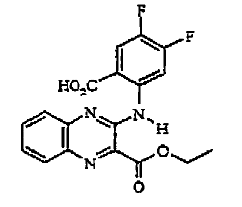 | 0,1187 | 381,5 | 0,4067 | 287,0 |
| 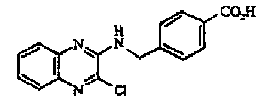 | 0,2348 | 333,0 | 0,2183 | 290,0 |
| | 0,2330 | 346,0 | | |
| 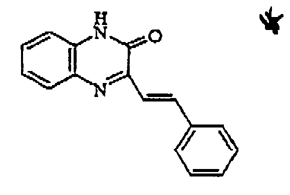 | 0,7324 | 388,5 | | |
| 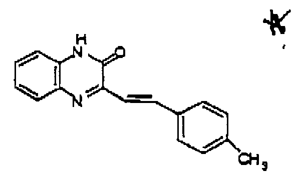 | 0,4660 | 336,0 | | |
| 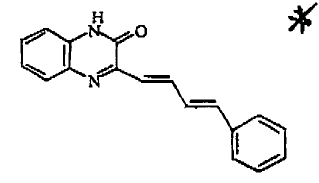 | | | 0,6004 | 281,5 |
| 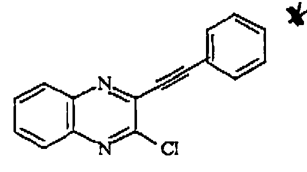 | 0,5539 | 366,0 | 0,6431 | 286,0 |
| | 0,5439 | 350,5 | | |
| 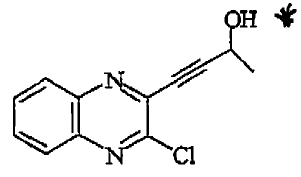 | 0,4653 | 352,0 | | |
| | 0,4557 | 338,5 | | |
| 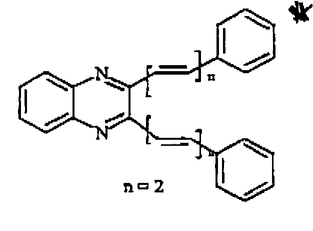 | 0,8610 | 363,5 | | |
| | 0,8531 | 327,5 | | |
| 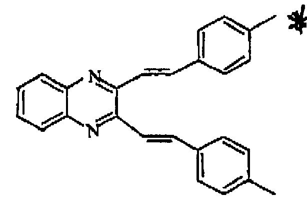 | | | 0,3327 | 316,0 |
| 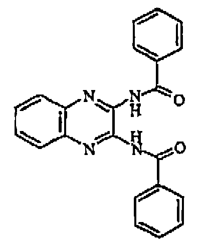 | 0,7643 | 368,0 | | |
| | 0,7984 | 352,0 | | |
| 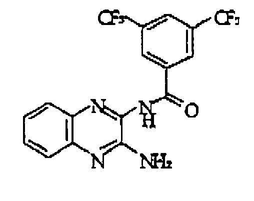 | 0,4130 | 354,0 | | |
| 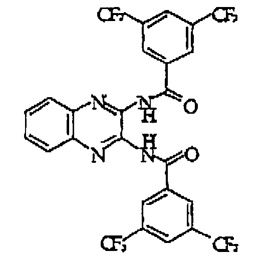 | 0,1734 | 349,0 | | |
| | 0,1826 | 340,0 | | |
| 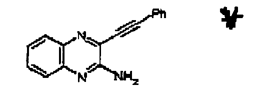 | 0,5137 | 395,3 | 0,3618 | 257,0 |
| | 0,5043 | 321,0 | 0,3968 | 202,0 |
| 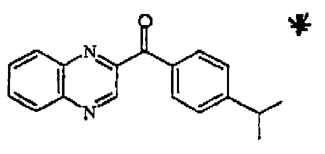 | - | - | - | - |
| 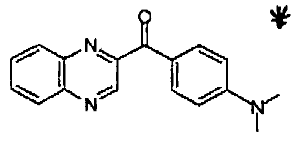 | 0,3405 | 387,0 | | |
| | 0,4828 | 322,0 | | |
| 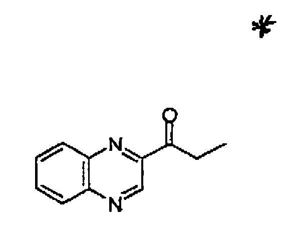 | | | 0,3236 | 307,0 |
| 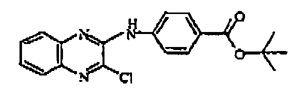 | 0,2677 | 391,0 | | |
| | 0,3342 | 373,0 | 0,4672 | 291,0 |
| | 0,5593 | 335,0 | | |
| | 0,5519 | 325,0 | | |
| | 0,2688 | 356,0 | | |
| 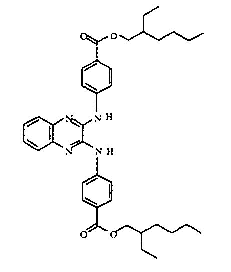 | 0,4647 | 336,0 | | |
| | 0,4269 | 323,0 | | |
| 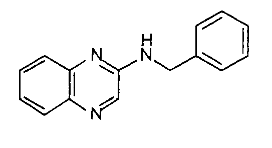 | 0,2602 | 364,0 | | |
| 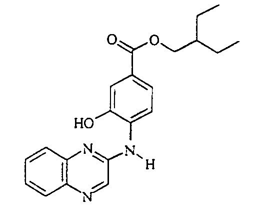 | 0,4059 | 381,0 | 0,5569 | 291,0 |
| | 0,5296 | 320,0 | | |
| 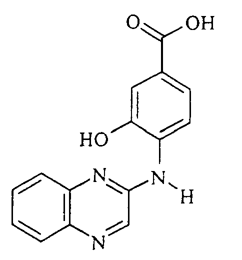 | 0,5594 | 381,0 | 0,7514 | 319,0 |
| | | | 0,8969 | 290,0 |
| 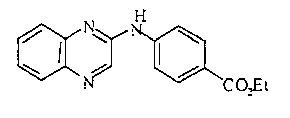 | 0,5414 | 375,0 | 1,2839 | 306,0 |
| 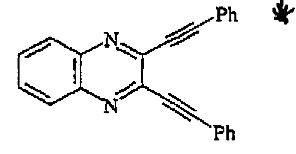 | 0,4443 | 385,0 | 0,8366 | 294,0 |
| 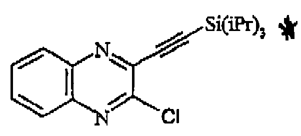 | 0,4936 | 355,0 | | |
| | 0,4868 | 340,0 | | |
| 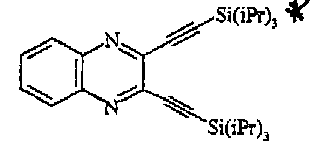 | 0,2594 | 375,0 | | |
| | 0,2348 | 358,0 | 1,0488 | 272,0 |
| 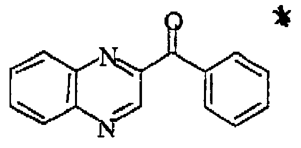 | | | 0,330 | 300,0 |
| 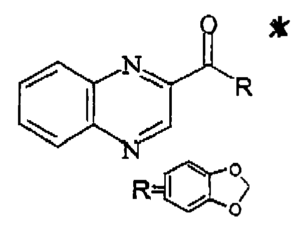 | | | 0,3349 | 336,0 |
| 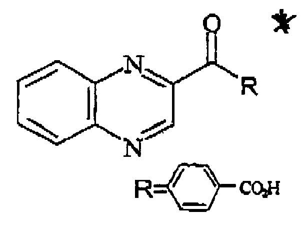 | 0,4206 | 307,0 | | |
| 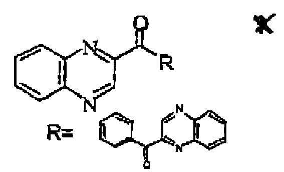 | | | 0,2798 | 317,0 |
| 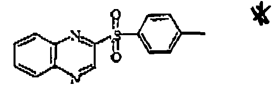 | 0,1595 | 321,0 | | |
| 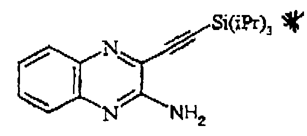 | 0,1707 | 390,0 | 0,1782 | 311,0 |
| 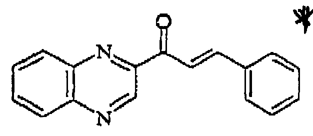 | 0,1731 | 329,0 | | |
| 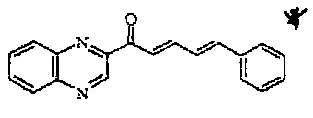 | 1,0497 | 365,0 | 0,6584 | 302,0 |
| 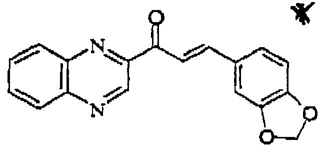 | 0,5101 | 381,0 | 0,4556 | 305,0 |
| 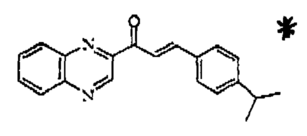 | 0,7731 | 343,0 | | |
| 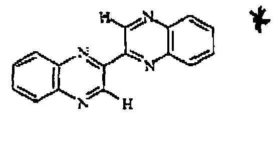 | 0,7687 | 341,0 | | |
| 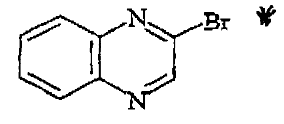 | 0,4424 | 321,0 | | |
| 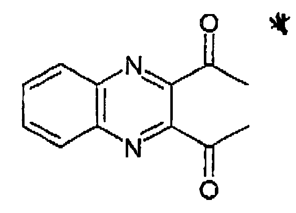 | | | 0,3011 | 310,0 |
| 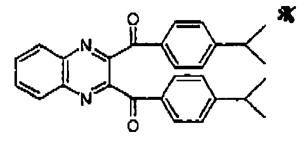 | | | 0,45 | 280,0 |
| 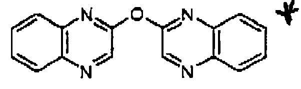 | 0,2587 | 320,0 | | |
| 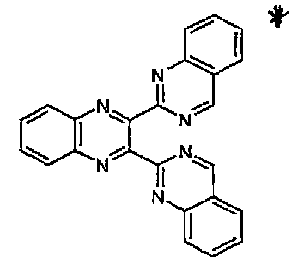 | 0,5570 | 335,0 | | |
| | 0,4203 | 333,0 | | |

| | | | | |
|---|---|---|---|---|
| * sind Vergleichsbeispiele | | | | |

### Anwendungsbeispiele

### Beispiel 1

### Herstellung eines erfindungsgemäßen Sonnenschutzsprays (O/W)

| A | | % |
|---|---|---|
| N-(2-Chinoxalinyl)-4-amino-benzoesäure-isopropylester | (1) | 1,00 |
| Eusolex 2292 (Art.-Nr. 105382) | (1) | 7,50 |
| (Octyl Methoxycinnamate) | | |
| Eusolex HMS (Art.-Nr. 111412) (Homosalate) | (1) | 7,00 |
| Volpo S-2 (Steareth-2) | (2) | 0,40 |
| Volpo S-10 (Steareth-10) | (2) | 0,80 |
| Pemulen TR-2 (Acrylate/C10-39Alkyl Acrylate Crosspolymer) | (3) | 0,18 |
| Hetester PHA (Propylene Glycol Isoceteth-3 Acetate) | (4) | 5,00 |
| Performa V 825 | (5) | 0,80 |
| (Synthetic Wax) | | |
| Dow Corning 200 (100cs) | (6) | 1,00 |
| (Dimethicone) | | |
| Oxynex K flüssig (Art.-Nr. 108324) | (1) | 0,10 |
| (PEG-8 (and) Tocopherol (and) Ascorbyl Palmitate (and) Ascorbic Acid (and) Citric Acid) | | |

| B | | % |
|---|---|---|
| Eusolex 232 (Art.-Nr. 105372) | (1) | 1,00 |
| (Phenylbenzimidazole Sulfonic Acid) | | |
| Triethanolamin (Art.-Nr. 108379) | (1) | 0,90 |
| Propandiol-1,2 (Art.-Nr. 107478) | (1) | 2,00 |
| Konservierungsmittel | | q.s. |
| Wasser, demineralisiert | ad | 100,00 |

### Herstellung:

Zur Neutralisierung von Eusolex 232 wird das Triethanolamin ins Wasser der Phase B gegeben und Eusolex 232 unter Rühren zugegeben. Nach vollständiger Lösung werden die restlichen Rohstoffe der Phase B zugegeben und auf 80°C erhitzt. Phase A bis auf das Pemulen zusammengegeben und auf 80 °C erhitzen. Pemulen in Phase A einrühren. Phase B unter Rühren langsam zu Phase A geben, homogenisieren und unter Rühren abkühlen.

### Bemerkunqen:

Als Konservierungsmittel werden verwendet:
0,05 % Propyl-4-hydroxybenzoat (Art. Nr. 107427)
0,15 % Methyl-4-hydroxybenzoat (Art. Nr. 106757)

### Bezugsquellen:

(1) Merck KGaA, Darmstadt
(2) Croda, Nettetal
(3) Goodrich, Neuss
(4) ROVI, Schlüchtern
(5) New Phase, NJ 08554
(6) Dow Corning, Wiesbaden

### Beispiel 2

Herstellung eines erfindungsgemäßen Sonnenschutzgels (O/W)

| A | | % |
|---|---|---|
| N-(2-Chinoxalinyl)-4-amino-benzoesäure-isopropylester | (1) | 1,00 |
| Eusolex 6300 (Art.-Nr. 5385) | (1) | 10,00 |
| Luvitol EHO | (2) | 2,00 |
| Dow Corning 200 (100 cs) | (3) | 2,00 |
| Sheabutter | (4) | 5,00 |
| Antaron V-220 | (5) | 2,00 |
| Oxynex K flüssig (Art.-Nr. 8324) | (6) | 1,00 |
| | | |

| B | | % |
|---|---|---|
| Eusolex 232 (Art.-Nr. 5372) | (1) | 0,75 |
| Tris(hydroxymethyl)-aminomethan (Art.-Nr. 8386) | (1) | 0,33 |
| Konservierungsmittel | | q.s. |
| Wasser, demineralisiert | | 20,00 |
| | | |

| C | | % |
|---|---|---|
| Tris(hydroxymethyl)-aminomethan (Art.-Nr. 8386) | (1) | 1,20 |
| Wasser, dem. | | 10,00 |

| D | | % |
|---|---|---|
| Pemulen TR-1 | (6) | 0,60 |
| Wasser, dem. | ad | 100,00 |

### Herstellung:

Für Phase D das Pemulen TR-1 im Wasser homogen dispergieren und unter Rühren die vorgelöste Phase C zugeben. Zur Neutralisierung von Eusolex 232 wird das Tris(hydroxymethyl)-aminomethan im Wasser der Phase B gelöst und das Eusolex 232 unter Rühren zugegeben. Nach vollständiger Lösung werden die restlichen Rohstoffe der Phase B zugegeben. Phase B in Phase C/D einrühren. Phase A unter Erwärmen lösen und in Phase B/C/D einrühren. Homogenisieren.

### Bemerkungen:

Als Konservierungsmittel werden eingesetzt:
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) Merck KGaA, Darmstadt
(2) BASF, Ludwigshafen
(3) Dow Corning, Düsseldorf
(4) H. Erhard Wagner, Bremen
(5) GAF, Frechen
(6) Goodrich, Neuss

### Beispiel 3

Herstellung einer Sonnenschutzlotion (W/O) mit UV-A/B-Schutz

| A | | % |
|---|---|---|
| N-(2-Chinoxalinyl)-4-amino-benzoesäure-isopropylester | (1) | 1,00 |
| Eusolex 2292 (Art.-Nr. 1.05382) | (1) | 3,00 |
| Eusolex 4360 (Art.-Nr. 1.05376) | (1) | 2,00 |
| (Benzophenone-3) | | |
| Dehymuls E | (2) | 6,00 |
| (Dicocoyl Pentyerythrityl Citrate (and) Sorbitan | | |
| Sesquioleate (and) Cera Alba (and) Aluminium Stearate) | | |
| Arlacel 989 | (3) | 1,00 |
| (PEG-7 Hydrogenated Castor Oil) | | |
| Bienenwachs (Art.-Nr. 1.11544) | (2) | 2,00 |
| Cetiol J 600 | (2) | 6,00 |
| (Oleyl Erucate) | | |
| Cetiol V | (2) | 6,00 |
| (Decyl Oleate) | | |
| CetiolOE | (2) | 5,00 |
| (Dicaprylyl Ether) | | |
| Dow Corning 200 (100 cs) | (4) | 1,00 |
| (Dimethicone) | | |
| | | |

| B | | % |
|---|---|---|
| Glycerin (etwa 87%) (Art.-Nr. 1.04091) | (1) | 5,00 |
| Magnesiumsulfat Heptahydrat (Art.-Nr. 1.05882) | (1) | 1,00 |
| Konservierungsmittel | | q.s. |
| Wasser, dem. | ad | 100,00 |

### Herstellung:

Phase B auf 80°C und Phase A auf 75 °C erhitzen. Phase B langsam in Phase A einrühren. Homogenisieren und unter Rühren abkühlen.

### Bemerkungen:

Als Konservierungsmittel sind enthalten:
0,05 % Propyl-4-hydroxybenzoat (Art. Nr. 1.07427)
0,15 % Methyl-4-hydroxybenzoat (Art. Nr. 1.06757)

### Bezugsquellen:

(1) Merck KGaA, Darmstadt
(2) Henkel KGaA, Düsseldorf
(3) ICI, Essen
(4) Dow Corning, Düsseldorf

### Beispiel 4

Herstellung einer Sonnenschutzlotion mit IR3535^{™}

| A | | % |
|---|---|---|
| N-(2-Chinoxalinyl)-4-amino-benzoesäure | (1) | 3,00 |
| Eusolex 6300 | (1) | 1,00 |
| (4-Methylbenzyliden Camphor) | | |
| IR 3535^{™} (Art.-Nr. 111887) | (1) | 10,00 |
| (Ethyl Butylacetylaminopropionate) | | |
| (-)-α-Bisabolol (Art.-Nr.130170) | (1) | 0,30 |
| Montanov 68 | (2) | 4,00 |
| (Cetearyl Alcohol (and) Cetearyl Glucoside) | | |
| Myritol 312 | (3) | 2,00 |
| (Carprylic/ apric Triglyceride) | | |
| Mirasil CM 5 | (4) | 2,00 |
| (Cyclomethicone) | | |
| Mirasil DM 350 | (4) | 1,00 |
| (Dimethicone) | | |
| | | |

| B | | % |
|---|---|---|
| Demin. Wasser | ad | 100,00 |
| Glycerin, 87%ig (Art.-Nr. 104091) | (1) | 3,00 |
| Konservierungsmittel | | q.s. |
| | | |

| C | | % |
|---|---|---|
| Rhodicare S | (4) | 0,50 |
| (Xanthan Gum) | | |

### Herstellung:

Phasen A und B getrennt auf 75°C erhitzen. C bei 75°C unter Rühren langsam zu B geben und rühren bis eine homogene Mischung entsteht. Anschließend A unter Rühren zu B/C geben und homogenisieren. Unter Rühren abkühlen.

### Bemerkungen:

Als Konservierungsmittel sind enthalten:
0,05% Propyl-4-hydroxybenzoat (Merck KGaA, Art.-Nr. 130173),
0,15% Methyl-4-hydroxybenzoat (Merck KGaA, Art.-Nr. 130174),
0,30% Germall 115 (ISP, Frechen)

### Bezugsquellen:

(1) Merck KGaA, Darmstadt
(2) Interorgana, Köln
(3) Henkel, KGaA, Düsseldorf
(4) Rhodia, Frankfurt

### Beispiel 5

Herstellung einer Sonnenschutzmilch mit IR3535^{™}

| A | | % |
|---|---|---|
| N-(2-Chinoxalinyl)-4-amino-benzoesäure | (1) | 1,00 |
| Eusolex 6300 | (1) | 1,00 |
| (4-Methylbenzyliden Camphor) | | |
| IR 3535^{™} (Art.-Nr. 111887) | (1) | 15,00 |
| (Ethyl Butylacetylaminopropionate) | | |
| Eusolex 2292 | (1) | 3,00 |
| (Octyl Methoxycinnamate) | | |
| Dow Corning 5225 C | (2) | 12,00 |
| (Cyclomethicone (and) Dimethicone Copolyol) | | |
| Dow Corning 345 | (2) | 5,00 |
| (Cyclomethicone) | | |
| Gilugel Sil 5 | (3) | 12,00 |
| (Cyclomethicone (and) Al/Mg Hydroxyde Stearate) | | |
| Solvent ID | (4) | 13,00 |
| (Isododecane) | | |
| Wiconol 14 | (5) | 2,50 |
| (Polyglyeryl-4 Oleate) | | |
| Bienenwachs, gebleicht (Art.-Nr. 111544) | (1) | 1,60 |
| Carnaubawachs | (6) | 0,40 |
| | | |

| B | | % |
|---|---|---|
| Demin. Wasser | ad | 100,00 |
| Propanediol-1,2 (Art.-Nr. 107478) | (1) | 2,00 |
| Natriumchlorid (Art.-Nr. 106400) | (1) | 2,00 |
| Konservierungsmittel | (1) | q.s. |
| | | |

| C | | % |
|---|---|---|
| Parfümöl Bariton (10607) | (7) | 0,30 |

### Herstellung:

Phasen A auf 80°C erhitzen, unter Rühren auf 30°C abkühlen. Phase B unter Rühren langsam zu Phase A geben, rühren bis eine homogene Mischung entsteht und homogenisieren. Phase C zugeben.

### Bemerkungen:

Als Konservierungsmittel ist enthalten:
0,20% Euxyl K400 (Schülke & Mayr, Norderstedt)

### Bezugsquellen:

(1) Merck KGaA, Darmstadt
(2) Dow Corning, Düsseldorf
(3) Nordmann, Rassmann GmbH&Co, Hamburg
(4) BP, Düsseldorf
(5) Witco Chemical, Frankfurt
(6) Aug. Schmidt Nachfolger, Bremen
(7) Haarmann & Reimer, Holzminden

## Patentansprüche

1. Verwendung von Chinoxalinderivaten der Formeln Ik und/oder II 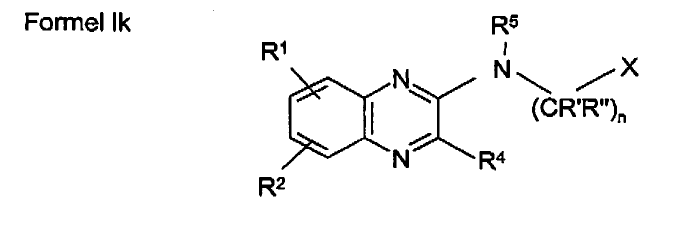 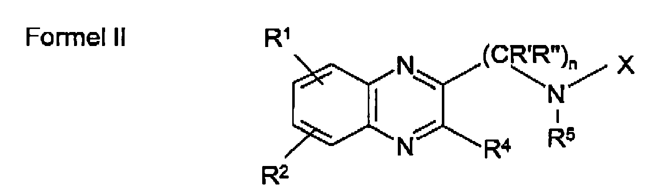 worin
R¹ und R² jeweils unabhängig voneinander H, Alkyl, Alkoxy, Alkenyl oder Alkinyl jeweils mit bis zu 20 C-Atomen, Cycloalkyl, Cycloalkoxy, Cycloalkenyl oder Bicyclische Systeme jeweils mit bis zu 10 C-Atomen, wobei in allen diesen Gruppen auch ein oder mehrere Wasserstoffatome durch Sub¹ substituiert und/oder eine oder zwei CH₂-Gruppen durch C=O ersetzt sein können und in den cyclischen Systemen 1 bis 3 Heteroatome wie S, N und/oder O enthalten sein können, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵ wasserlöslich machende Substituenten, ausgewählt aus der Gruppe bestehend aus Carboxylat-, Sulfonat- oder Ammoniumresten, COR⁵, COOR⁵, CON⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Het, -NHHet,-OHet oder -(CR⁵R⁶)ₙ-Het,
R¹ und R² zusammen auch mit Kohlenstoffatomen, an die sie gebunden sind, gemeinsam einen ungesättigten, teilweise oder vollständig gesättigten 4-, 5-, 6- oder 7-Ring bilden können, der gegebenenfalls Heteroatome wie S, N und/oder O enthalten kann, weiter anelliert und/oder auch ein- oder mehrfach substituiert sein kann,
Sub¹ Hal, Hydroxy, Cyano, Amino, Nitro, C₁-C₄ -Alkylamino, C₁-C₄-Dialkylamino, C₁-C₄-Alkyl oder C₁-C₄ -Alkoxy, COOH oder COOAlkyl,
Hal Fluor, Chlor, Brom, Jod,
n 0, 1, 2, 3 oder 4,
R⁵, R⁶ jeweils unabhängig voneinander H, Alkyl, Alkenyl, Alkinyl, jeweils mit bis zu 20 C-Atomen, Cycloalkyl, Cycloalkenyl, Bicyclische Systeme, jeweils mit bis zu 10 C-Atomen, wobei diese Reste bis dreifach durch Sub¹ substituiert vorliegen und/oder ein oder zwei CH₂-Gruppen durch C=O ersetzt sein können, und die cyclischen Systeme auch 1 bis 3 Heteroatome wie S, N, und/oder O enthalten können, -(CR'R")ₙ-Ar oder -(CR'R")ₙ-Het,
R und R" jeweils unabhängig voneinander H oder C₁-C₄-Alkyl, wobei auch ein oder zwei CH₂-Gruppen durch C=O ersetzt sein können,
X Ar oder Het,
Ar ein unsubstituierter oder ein- oder mehrfach substituierter aromatischer Ring oder kondensierte Ringsysteme mit 6 bis 18 C-Atomen, worin auch ein oder zwei CH-Gruppen durch C=O ersetzt sein können,
Het ein unsubstituierter oder ein-oder mehrfach substituierter heteroaromatischer Ring mit 5 bis 7 Ringgliedern oder ein kondensiertes Ringsystem, wobei als Heteroatome ein oder mehrere N-, S- und/oder O-Atome enthalten sind und worin auch ein oder zwei CH-Gruppen in α- oder β-Position zu den Heteroatomen durch C=O ersetzt sein können,
R⁴ H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome wie S, N, und/oder O enthalten können, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵ wasserlöslich machende Substituenten, ausgewählt aus der Gruppe bestehend aus Carboxylat-, Sulfonat- oder Ammoniumresten, COR⁵, COOR⁵, CONR⁵R⁶, CN, O=S(-R⁵)=O, O=S(-OR⁵)=O, O=S(-NR⁵R⁶)=O, R⁷OP(-OR⁸)=O, OAr, -(CR⁵R⁶)ₙ-Ar, -Het, -NMHet,-OHet oder -(CR⁵R⁶)ₙ-Het,
Sub² Hal, Hydroxy, Cyano, Amino, Nitro, C₁-C₄-Alkyl oder C₁-C₄ Alkoxy, COR⁵, COOR⁵, OAr, OHet, -(CR⁵R⁶)ₙ-Ar oder -(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁶)=O oder R⁷OP(-OR⁸)=O,
R⁷ und R⁸ jeweils unabhängig voneinander H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome ausgewählt aus N, und/oder O enthalten können, bedeuten,
als UV-Filtersubstanzen.

2. Verwendung von Chinoxalinderivaten der Formeln Ik und/oder II 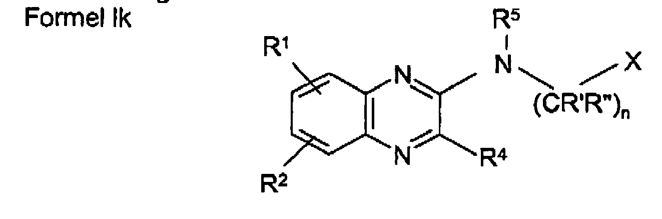 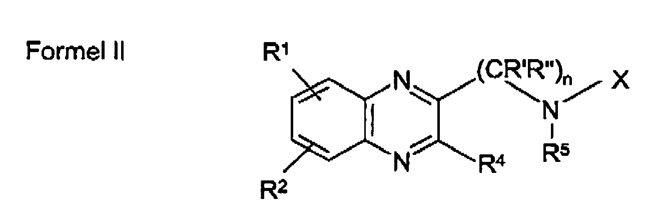 worin
R¹und R² jeweils unabhängig voneinander H, Alkyl, Alkoxy, Alkenyl oder Alkinyl jeweils mit bis zu 20 C-Atomen, Cycloalkyl, Cycloalkoxy, Cycloalkenyl oder Bicyclische Systeme jeweils mit bis zu 10 C-Atomen, wobei in allen diesen Gruppen auch ein oder mehrere Wasserstoffatome durch Sub¹ substituiert und/oder eine oder zwei CH₂-Gruppen durch C=O ersetzt sein können und in den cyclischen Systemen 1 bis 3 Heteroatome wie S, N und/oder O enthalten sein können, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵ wasserlöslich machende Substituenten, ausgewählt aus der Gruppe bestehend aus Carboxylat-, Sulfonat- oder Ammoniumresten, COR⁵, COOR⁵, CON⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ₋Ar, -Het, -NHHet,-OHet oder -(CR⁵R⁶)ₙ-Het,
R¹ und R² zusammen auch mit Kohlenstoffatomen, an die sie gebunden sind, gemeinsam einen ungesättigten, teilweise oder vollständig gesättigten 4-, 5-, 6- oder 7-Ring bilden können, der gegebenenfalls Heteroatome wie S, N und/oder O enthalten kann, weiter anelliert und/oder auch ein- oder mehrfach substituiert sein kann,
Sub¹ Hal, Hydroxy, Cyano, Amino, Nitro, C₁-C₄ -Alkylamino, C₁-C₄-Dialkylamino, C₁-C₄-Alkyl oder C₁-C₄ Alkoxy, COOH oder COOAlkyl,
Hal Fluor, Chlor, Brom, Jod,
n 0, 1, 2, 3 oder 4,
R⁵, R⁶ jeweils unabhängig voneinander H, Alkyl, Alkenyl, Alkinyl, jeweils mit bis zu 20 C-Atomen, Cycloalkyl, Cycloalkenyl, Bicyclische Systeme, jeweils mit bis zu 10 C-Atomen, wobei diese Reste bis dreifach durch Sub¹ substituiert vorliegen und/oder ein oder zwei CH₂-Gruppen durch C=O ersetzt sein können, und die cyclischen Systeme auch 1 bis 3 Heteroatome wie S, N, und/oder O enthalten können, -(CR'R")ₙ-Ar oder -(CR'R")ₙ-Het,
R' und R" jeweils unabhängig voneinander H oder C₁-C₄-Alkyl, wobei auch ein oder zwei CH₂-Gruppen durch C=O ersetzt sein können,
X Ar oder Het,
Ar ein unsubstituierter oder ein- oder mehrfach substituierter aromatischer Ring oder kondensierte Ringsysteme mit 6 bis 18 C-Atomen, worin auch ein oder zwei CH-Gruppen durch C=O ersetzt sein können,
Het ein unsubstituierter oder ein-oder mehrfach substituierter heteroaromatischer Ring mit 5 bis 7 Ringgliedern oder ein kondensiertes Ringsystem, wobei als Heteroatome ein oder mehrere N-, S- und/oder O-Atome enthalten sind und worin auch ein oder zwei CH-Gruppen in α- oder β-Position zu den Heteroatomen durch C=O ersetzt sein können,
R⁴ H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome wie S, N, und/oder O enthalten können, Hal, OH, NO₂, -(CR⁵R⁶)ₙ-NR⁵R⁶, -(CR⁵R⁶)ₙ-N=CR⁵R⁶, -(CR⁵R⁶)ₙ-C⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵ wasserlöslich machende Substituenten, ausgewählt aus der Gruppe bestehend aus Carboxylat-, Sulfonat- oder Ammoniumresten, COR⁵, COOR⁵, CONR⁵R⁶, CN, O=S(-R⁵)=O, O=S(-OR⁵)=O, O=S(-NR⁵R⁶)=O, R⁷OP(-OR⁸)=O, OAr, -(CR⁵R⁶)ₙ-Ar, -Het, -NHHet,-OHet oder -(CR⁵R⁶)ₙ-Het,
Sub² Hal, Hydroxy, Cyano, Amino, Nitro, C₁-C₄-Alkyl oder C₁-C₄ -Alkoxy, COR⁵, COOR⁵, OAr, OHet, -(CR⁵R⁶)ₙ-Ar oder -(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁹)=O oder R⁷OP(-OR⁸)=O,
R⁷ und R⁸ jeweils unabhängig voneinander H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome ausgewählt aus N, und/oder O enthalten können, bedeuten,
zur Herstellung pharmazeutischer Zubereitungen zum Schutz der menschlichen Haut oder menschlicher Haare gegen Sonnenstrahlen, allein oder zusammen mit an sich für pharmazeutische Zubereitungen bekannten, im UV-Bereich absorbierenden Verbindungen.

3. Verwendung von Verbindungen der Formeln Ik und/oder II, in der die Variablen die Bedeutung nach Anspruch 1 oder 2 haben, als UV-Stabilisatoren in kosmetischen Zubereitungen.

4. Verbindungen aus der Gruppe
N-(2-Chinoxalinyl)-4-amino-benzoesäureisopropylester,
N,N-Benzoyl-(2-chinoxalinyl)-4-amino-benzoesäure;
N-(2-Chinoxalinyl)-4-aminomethyl-benzoesäure;
N-(2-Chinoxalinyl)-2-amino-4,5-dimethoxy-benzoesäure;
N-(2-Chinoxalinyl)-2-amino-pyrimidin;
N-(2-Chinoxalinyl)-2-amino-benzophenon;
N-(2-Chinoxalinyl)-4-amino-anisol;
N-(2-Chinoxalinyl)-3,4,5-trimethoxy-anilin;
N-[Bis-(2-chinoxalinyl)]-2,4,6-trifluoro-anilin;
N-[2-(3-Phenyl)-chinoxalinyl]-4-amino-benzoesäure.

5. Lichtschutzmittel enthaltende kosmetische oder pharmazeutische Zubereitungen zum Schutz der menschlichen Epidermis oder menschlichen Haare gegen UV-Licht im Bereich von 280 bis 400 nm, **dadurch gekennzeichnet, dass** sie in einem kosmetisch geeigneten Träger, allein oder zusammen mit an sich für kosmetische Zubereitungen bekannten im UV-Bereich absorbierenden Verbindungen, als photostabile UV-Filter wirksame Mengen von Verbindungen der Formeln Ik und/oder II enthalten, in der die Variablen die Bedeutung nach Anspruch 1 oder 2 haben.

6. Kosmetische oder pharmazeutische Zubereitung nach Anspruch 5, wobei die Zubereitung einen oder mehrere UV-Filter enthält, die vorzugsweise ausgewählt sind aus der Gruppe, die 3-(4'-Methylbenzyliden)-dI-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

7. Kosmetische oder pharmazeutische Zubereitung nach Anspruch 5 oder 6, **dadurch gekennzeichnet, dass** ein oder mehrere Antixidantien und/oder mindestens eine der Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) und/oder mindestens ein Aryloxim, vorzugsweise 2-Hydroxy5-methyllaurophenonoxim und/oder ein Coumaranonderivat, vorzugsweise 4,6,3',4'-Tetrahydroxybenzylcoumaranon-3 oder dessen Trisulfat enthalten sind.

## Claims

1. Use of quinoxaline derivatives of the formulae Ik and/or II 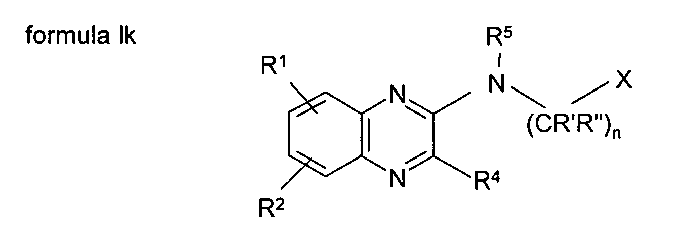 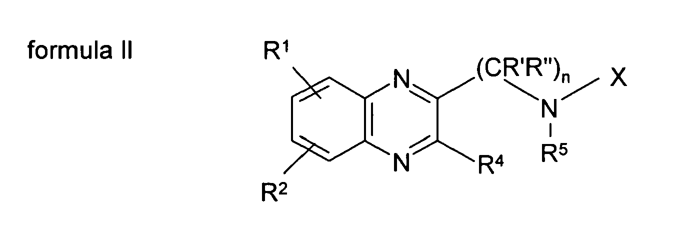 in which
R¹and R² each, independently of one another, denote H, alkyl, alkoxy, alkenyl or alkynyl, each having up to 20 C atoms, cycloalkyl, cycloalkoxy, cycloalkenyl or bicyclic systems, each having up to 10 C atoms, where, in each of these groups, one or more hydrogen atoms may also be substituted by Sub¹ and/or one or two CH₂ groups may be replaced by C=O, and the cyclic systems may contain 1 to 3 heteroatoms, such as S, N and/or O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵ NR⁵-SO-R⁶, -SO-R⁵, water-solubilising substituents selected from the group consisting of carboxylate, sulfonate or ammonium radicals, COR⁵, COOR⁵, CONR⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Het, -NHHet, -OHet or -(CR⁵R⁶)ₙ-Het,
R¹ and R² together, also with carbon atoms to which they are bonded, may jointly form an unsaturated, partially or fully saturated 4-, 5-, 6- or 7-membered ring, which may optionally contain heteroatoms, such as S, N and/or O, may be further fused and/or may also be mono- or polysubstituted,
Sub¹ denotes Hal, hydroxyl, cyano, amino, nitro, C₁-C₄-alkyl-amino, C₁-C₄-dialkylamino, C₁-C₄-alkyl or C₁-C₄-alkoxy, COOH or COO-alkyl,
Hal denotes fluorine, chlorine, bromine, iodine,
n denotes 0, 1, 2, 3 or 4,
R⁵, R⁶ each, independently of one another, denote H, alkyl, alkenyl, alkynyl, each having up to 20 C atoms, cycloalkyl, cycloalkenyl, bicyclic systems, each having up to 10 C atoms, where these radicals may be up to trisubstituted by Sub¹ and/or one or two CH₂ groups may be replaced by C=O, and the cyclic systems may also contain 1 to 3 heteroatoms, such as S, N and/or O, -(CR'R")ₙ-Ar or -(CR'R")ₙ-Het,
R and R" each, independently of one another, denote H or C₁-C₄-alkyl, where one or two CH₂ groups may also be replaced by C=O,
X denotes Ar or Het,
Ar denotes an unsubstituted or mono- or polysubstituted aromatic ring or condensed ring systems having 6 to 18 C atoms, in which, in addition, one or two CH groups may be replaced by C=O,
Het denotes an unsubstituted or mono- or polysubstituted heteroaromatic ring having 5 to 7 ring members or a condensed ring system, in which one or more N, S and/or O atoms are present as heteroatoms and in which, in addition, one or two CH groups in the α- or β-position to the heteroatoms may be replaced by C=O,
R⁴ denotes H, alkyl, alkoxy, alkenyl, alkynyl, each having up to 20 C atoms, cycloalkyl, cycloalkoxy, cycloalkenyl, bicyclic systems, each having up to 10 C atoms, where these radicals may be up to trisubstituted by Sub² and/or one or two CH₂ groups may be replaced by C=O and where the cyclic systems may also contain 1 to 3 heteroatoms, such as S, N and/or O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵, water-solubilising substituents selected from the group consisting of carboxylate, sulfonate or ammonium radicals, COR⁵, COOR⁵, CONR⁵R⁶, CN, O=S(-R⁵)=O_{,} O=S(-OR⁵)=O_{,} O=S(-NR⁵R⁶)=O, R⁷OP(-OR⁸)=O, OAr, -(CR⁵R⁶)ₙ-Ar, -Het, -NHHet, -OHet or -(CR⁵R⁶)ₙ-Het,
Sub² denotes Hal, hydroxyl, cyano, amino, nitro, C₁-C₄-alkyl or C₁-C₄-alkoxy, COR⁵, COOR⁵, OAr, OHet, -(CR⁵R⁶)ₙ-Ar or -(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁶)=O or R⁷OP(-OR⁸)=O,
R⁷ and R⁸ each, independently of one another, denote H, alkyl, alkoxy, alkenyl, alkynyl, each having up to 20 C atoms, cycloalkyl, cycloalkoxy, cycloalkenyl, bicyclic systems, each having up to 10 C atoms, where these radicals may be up to trisubstituted by Sub² and/or one or two CH₂ groups may be replaced by C=O and where the cyclic systems may also contain 1 to 3 heteroatoms selected from N and/or O,
as UV filter substances.

2. Use of quinoxaline derivatives of the formulae Ik and/or II 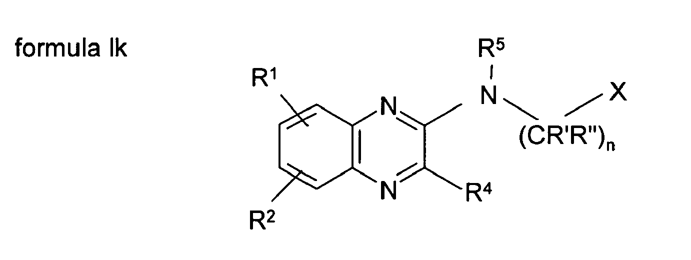 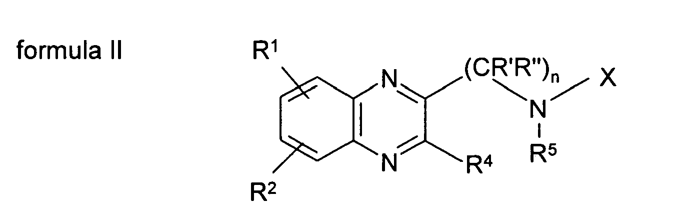 in which
R¹ and R² each, independently of one another, denote H, alkyl, alkoxy, alkenyl or alkynyl, each having up to 20 C atoms, cycloalkyl, cycloalkoxy, cycloalkenyl or bicyclic systems, each having up to 10 C atoms, where, in each of these groups, one or more hydrogen atoms may also be substituted by Sub¹ and/or one or two CH₂ groups may be replaced by C=O, and the cyclic systems may contain 1 to 3 heteroatoms, such as S, N and/or O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵, water-solubilising substituents selected from the group consisting of carboxylate, sulfonate or ammonium radicals, COR⁵, COOR⁵, CONR⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Het, -NHHet, -OHet or -(CR⁵R⁶)ₙ-Het,
R¹ and R² together, also with carbon atoms to which they are bonded, may jointly form an unsaturated, partially or fully saturated 4-, 5-, 6- or 7-membered ring, which may optionally contain heteroatoms, such as S, N and/or O, may be further fused and/or may also be mono- or polysubstituted,
Sub¹ denotes Hal, hydroxyl, cyano, amino, nitro, C₁-C₄-alkylamino, C₁-C₄-dialkylamino, C₁-C₄-alkyl or C₁-C₄-alkoxy, COOH or COO-alkyl,
Hal denotes fluorine, chlorine, bromine, iodine,
n denotes 0, 1, 2, 3 or 4,
R⁵, R⁶ each, independently of one another, denote H, alkyl, alkenyl, alkynyl, each having up to 20 C atoms, cycloalkyl, cycloalkenyl, bicyclic systems, each having up to 10 C atoms, where these radicals may be up to trisubstituted by Sub¹ and/or one or two CH₂ groups may be replaced by C=O, and the cyclic systems may also contain 1 to 3 heteroatoms, such as S, N and/or O, -(CR'R")ₙ-Ar or -(CR'R")ₙ-Het,
R' and R" each, independently of one another, denote H or C₁-C₄-alkyl, where one or two CH₂ groups may also be replaced by C=O,
X denotes Ar or Het,
Ar denotes an unsubstituted or mono- or polysubstituted aromatic ring or condensed ring systems having 6 to 18 C atoms, in which, in addition, one or two CH groups may be replaced by C=O,
Het denotes an unsubstituted or mono- or polysubstituted heteroaromatic ring having 5 to 7 ring members or a condensed ring system, in which one or more N, S and/or O atoms are present as heteroatoms and in which, in addition, one or two CH groups in the α- or β-position to the heteroatoms may be replaced by C=O,
R⁴ denotes H, alkyl, alkoxy, alkenyl, alkynyl, each having up to 20 C atoms, cycloalkyl, cycloalkoxy, cycloalkenyl, bicyclic systems, each having up to 10 C atoms, where these radicals may be up to trisubstituted by Sub² and/or one or two CH₂ groups may be replaced by C=O and where the cyclic systems may also contain 1 to 3 heteroatoms, such as S, N and/or O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵, water-solubilising substituents selected from the group consisting of carboxylate, sulfonate or ammonium radicals, COR⁵, COOR⁵, CONR⁵R⁶, CN, O=S(-R⁵)=O, O=S(-OR⁵)=O, O=S(-NR⁵R⁶)=O, R⁷OP(-OR⁸)=O, OAr, -(CR⁵R⁶)ₙ-Ar, -Het, -NHHet, -OHet or -(CR⁵R⁶)ₙ-Het,
Sub² denotes Hal, hydroxyl, cyano, amino, nitro, C₁-C₄-alkyl or C₁-C₄-alkoxy, COR⁵, COOR⁵, OAr, OHet, -(CR⁵R⁶)ₙ-Ar or -(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁶)=O or R⁷OP(-OR⁸)=O,
R⁷ and R⁸ each, independently of one another, denote H, alkyl, alkoxy, alkenyl, alkynyl, each having up to 20 C atoms, cycloalkyl, cycloalkoxy, cycloalkenyl, bicyclic systems, each having up to 10 C atoms, where these radicals may be up to trisubstituted by Sub² and/or one or two CH₂ groups may be replaced by C=O and where the cyclic systems may also contain 1 to 3 heteroatoms selected from N and/or O,
for the preparation of pharmaceutical compositions for protection of the human skin or human hair against solar radiation, alone or together with UV-absorbent compounds known per se for pharmaceutical compositions.

3. Use of compounds of the formulae Ik and/or II, in which the variables have the meaning according to Claim 1 or 2, as UV stabilisers in cosmetic compositions.

4. Compounds from the group
isopropyl N-(2-quinoxalinyl)-4-aminobenzoate;
N,N-benzoyl-(2-quinoxalinyl)-4-aminobenzoic acid;
N-(2-quinoxalinyl)-4-aminomethylbenzoic acid;
N-(2-quinoxalinyl)-2-amino-4,5-dimethoxybenzoic acid;
N-(2-quinoxalinyl)-2-aminopyrimidine;
N-(2-quinoxalinyl)-2-aminobenzophenone;
N-(2-quinoxalinyl)-4-aminoanisole;
N-(2-quinoxalinyl)-3,4,5-trimethoxyaniline;
N-[bis(2-quinoxalinyl)]-2,4,6-trifluoroaniline;
N-[2-(3-phenyl)quinoxalinyl]-4-aminobenzoic acid.

5. Light-protection composition comprising cosmetic or pharmaceutical compositions for protection of the human epidermis or human hair against UV light in the range from 280 to 400 nm, **characterised in that** they comprise, in a cosmetically suitable vehicle, alone or together with UV-absorbent compounds known per se for cosmetic compositions, as photostable UV filters, effective amounts of compounds of the formulae Ik and/or II, in which the variables have the meaning according to Claim 1 or 2.

6. Cosmetic or pharmaceutical composition according to Claim 5, where the composition comprises one or more UV filters which are preferably selected from the group comprising 3-(4'-methylbenzylidene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and potassium, sodium and triethanolamine salts thereof.

7. Cosmetic or pharmaceutical composition according to Claim 5 or 6, **characterised in that** one or more antioxidants and/or at least one of the pyrimidinecarboxylic acids ectoin ((S)-1,4,5,6-tetrahydro-2-methyl-4-pyrimidinecarboxylic acid) and hydroxyectoin ((S,S)-1,4,5,6-tetrahydro-5-hydroxy-2-methyl-4-pyrimidinecarboxylic acid) and/or at least one aryl oxime, preferably 2-hydroxy-5-methyllaurophenone oxime, and/or a coumaranone derivative, preferably 4,6,3',4'-tetrahydroxybenzyl-3-coumaranone or the trisulfate thereof, are present.

## Revendications

1. Utilisation de dérivés de quinoxaline de formules Ik et/ou II 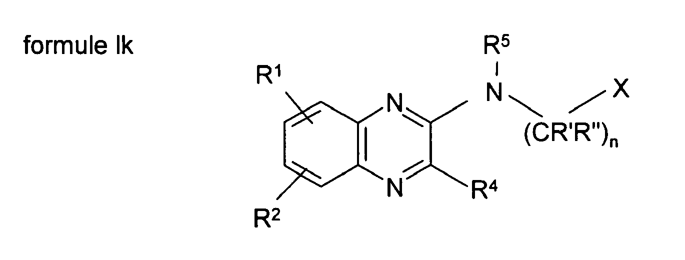 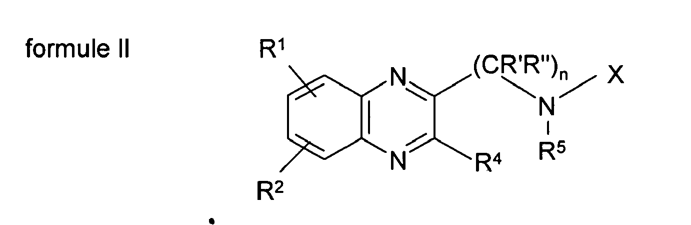 dans lesquelles
R¹ et R² désignent chacun, indépendamment l'un de l'autre, H, alkyle, alcoxy, alcényle ou alcynyle, ayant chacun jusqu'à 20 atomes de C, cycloalkyle, cycloalcoxy, cycloalcényle ou des systèmes bicycliques, ayant chacun jusqu'à 10 atomes de C, où, dans chacun de ces groupements, un ou plusieurs atomes d'hydrogène peuvent également être substitués par Sub¹ et/ou un ou deux groupements CH₂ peuvent être remplacés par C=O, et les systèmes cycliques peuvent contenir de 1 à 3 hétéroatomes, tels que S, N et/ou O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵, des substituants hydrosolubilisants choisis parmi le groupe constitué par les radicaux carboxylate, sulfonate ou ammonium, COR⁵, COOR⁵, CONR⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Hét, -NHHét, -OHét ou -(CR⁵R⁶)ₙ-Hét,
R¹ et R², conjointement avec les atomes de carbone auxquels ils sont fixés, peuvent former ensemble un cycle insaturé, partiellement ou totalement saturé de 4, 5, 6 ou 7 chaînons, pouvant éventuellement contenir des hétéroatomes, tels que S, N et/ou O, pouvant être en outre condensé et/ou pouvant également être mono- ou polysubstitué,
Sub¹ désigne Hal, hydroxyle, cyano, amino, nitro, C₁-C₄-alkylamino, C₁-C₄-dialkylamino, C₁-C₄-alkyle ou C₁-C₄-alcoxy, COOH ou COO-alkyle,
Hal désigne fluor, chlore, brome, iode,
n désigne 0, 1, 2, 3 ou 4,
R⁵, R⁶ désignent chacun, indépendamment l'un de l'autre, H, alkyle, alcényle, alcynyle, ayant chacun jusqu'à 20 atomes de C, cycloalkyle, cycloalcényle, des systèmes bicycliques, ayant chacun jusqu'à 10 atomes de C, où ces radicaux peuvent être jusqu'à trisubstitués par Sub¹ et/ou un ou deux groupements CH₂ peuvent être remplacés par C=O, et les systèmes cycliques peuvent également contenir de 1 à 3 hétéroatomes, tels que S, N et/ou O, -(CR'R")ₙ-Ar ou -(CR'R")ₙ-Hét,
R' et R" désignent chacun, indépendamment l'un de l'autre, H ou C₁-C₄-alkyle, où un ou deux groupements CH₂ peuvent également être remplacés par C=O,
X désigne Ar ou Hét,
Ar désigne un cycle aromatique non substitué ou mono- ou polysubstitué ou des noyaux condensés ayant de 6 à 18 atomes de C, où, en outre, un ou deux groupements CH peuvent être remplacés par C=O,
Hét désigne un cycle hétéroaromatique non substitué ou mono- ou polysubstitué ayant de 5 à 7 chaînons ou un noyau condensé, où un ou plusieurs atomes de N, S et/ou O sont présents comme hétéroatomes et où, en outre, un ou deux groupements CH en position α ou β par rapport aux hétéroatomes peuvent être remplacés par C=O,
R⁴ désigne H, alkyle, alcoxy, alcényle, alcynyle, ayant chacun jusqu'à 20 atomes de C, cycloalkyle, cycloalcoxy, cycloalcényle, des systèmes bicycliques, ayant chacun jusqu'à 10 atomes de C, où ces radicaux peuvent être jusqu'à trisubstitués par Sub² et/ou un ou deux groupements CH₂ peuvent être remplacés par C=O et où les systèmes cycliques peuvent également contenir de 1 à 3 hétéroatomes, tels que S, N et/ou O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵**,** NR⁵-SO-R⁶, -SO-R⁵, des substituants hydrosolubilisants choisis parmi le groupe constitué par les radicaux carboxylate, sulfonate ou ammonium, COR⁵, COOR⁵, CONR⁵R⁶, CN, O=S(-R⁵)=O, O=S(-OR⁵)=O, O=S(-NR⁵R⁶)=O, R⁷OP(-OR⁸)=O, OAr, -(CR⁵R⁶)ₙ-Ar, -Hét, -NHHét, -OHét ou -(CR⁵R⁶)ₙ-Hét,
Sub² désigne Hal, hydroxyle, cyano, amino, nitro, C₁-C₄-alkyle ou C₁-C₄-alcoxy, COR⁵, COOR⁵, OAr, OHét, -(CR⁵R⁶)ₙ-Ar ou -(CR⁵R⁶)ₙ-Hét, -(CR⁵R⁶)ₙ-NR⁵R⁶, CONR⁵R⁶, CN, O=S(-R⁵)=O, O=S(-OR⁵)=O, O=S(-NR⁵R⁶)=O ou R⁷OP(-OR⁸)=O,
R⁷ et R⁸ désignent chacun, indépendamment l'un de l'autre, H, alkyle, alcoxy, alcényle, alcynyle, ayant chacun jusqu'à 20 atomes de C, cycloalkyle, cycloalcoxy, cycloalcényle, des systèmes bicycliques, ayant chacun jusqu'à 10 atomes de C, où ces radicaux peuvent être jusqu'à trisubstitués par Sub² et/ou un ou deux groupements CH₂ peuvent être remplacés par C=O et où les systèmes cycliques peuvent également contenir de 1 à 3 hétéroatomes choisis parmi N et/ou O,
comme substances filtres UV.

2. Utilisation de dérivés de quinoxaline de formules Ik et/ou II 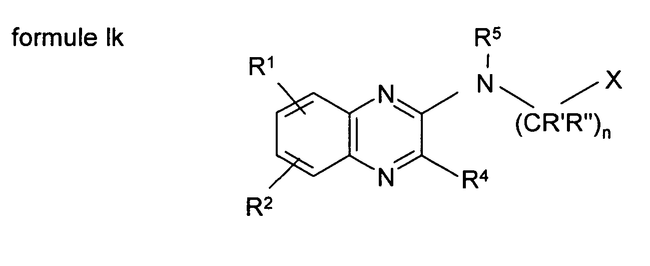 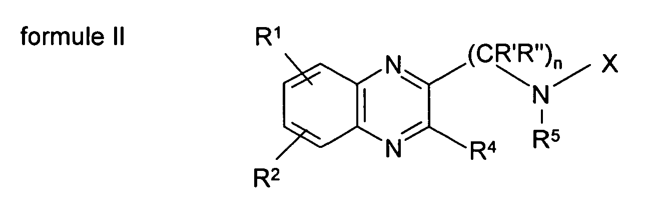 dans lesquelles
R¹ et R² désignent chacun, indépendamment l'un de l'autre, H, alkyle, alcoxy, alcényle ou alcynyle, ayant chacun jusqu'à 20 atomes de C, cycloalkyle, cycloalcoxy, cycloalcényle ou des systèmes bicycliques, ayant chacun jusqu'à 10 atomes de C, où, dans chacun de ces groupements, un ou plusieurs atomes d'hydrogène peuvent également être substitués par Sub¹ et/ou un ou deux groupements CH₂ peuvent être remplacés par C=O, et les systèmes cycliques peuvent contenir de 1 à 3 hétéroatomes, tels que S, N et/ou O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵, des substituants hydrosolubilisants choisis parmi le groupe constitué par les radicaux carboxylate, sulfonate ou ammonium, COR⁵, COOR⁵, CONR⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Hét, -NHHét, -OHét ou -(CR⁵R⁶)ₙ-Hét,
R¹ et R², conjointement avec les atomes de carbone auxquels ils sont fixés, peuvent former ensemble un cycle insaturé, partiellement ou totalement saturé de 4, 5, 6 ou 7 chaînons, pouvant éventuellement contenir des hétéroatomes, tels que S, N et/ou O, pouvant être en outre condensé et/ou pouvant également être mono- ou polysubstitué,
Sub¹ désigne Hal, hydroxyle, cyano, amino, nitro, C₁-C₄-alkylamino, C₁-C₄-dialkylamino, C₁-C₄-alkyle ou C₁-C₄-alcoxy, COOH ou COO-alkyle,
Hal désigne fluor, chlore, brome, iode,
n désigne 0, 1, 2, 3 ou 4,
R⁵, R⁶ désignent chacun, indépendamment l'un de l'autre, H, alkyle, alcényle, alcynyle, ayant chacun jusqu'à 20 atomes de C, cycloalkyle, cycloalcényle, des systèmes bicycliques, ayant chacun jusqu'à 10 atomes de C, où ces radicaux peuvent être jusqu'à trisubstitués par Sub¹ et/ou un ou deux groupements CH₂ peuvent être remplacés par C=O, et les systèmes cycliques peuvent également contenir de 1 à 3 hétéroatomes, tels que S, N et/ou O, -(CR'R")ₙ-Ar ou -(CR'R")ₙ-Hét,
R et R" désignent chacun, indépendamment l'un de l'autre, H ou C₁-C₄-alkyle, où un ou deux groupements CH₂ peuvent également être remplacés par C=O,
X désigne Ar ou Hét,
Ar désigne un cycle aromatique non substitué ou mono- ou polysubstitué ou des noyaux condensés ayant de 6 à 18 atomes de C, où, en outre, un ou deux groupements CH peuvent être remplacés par C=O,
Hét désigne un cycle hétéroaromatique non substitué ou mono- ou polysubstitué ayant de 5 à 7 chaînons ou un noyau condensé, où un ou plusieurs atomes de N, S et/ou O sont présents comme hétéroatomes et où, en outre, un ou deux groupements CH en position α ou β par rapport aux hétéroatomes peuvent être remplacés par C=O,
R⁴ désigne H, alkyle, alcoxy, alcényle, alcynyle, ayant chacun jusqu'à 20 atomes de C, cycloalkyle, cycloalcoxy, cycloalcényle, des systèmes bicycliques, ayant chacun jusqu'à 10 atomes de C, où ces radicaux peuvent être jusqu'à trisubstitués par Sub² et/ou un ou deux groupements CH₂ peuvent être remplacés par C=O et où les systèmes cycliques peuvent également contenir de 1 à 3 hétéroatomes, tels que S, N et/ou O, Hal, OH, NO₂, -(CR⁵R⁶)ₙ-NR⁵R⁶, -(CR⁵R⁶)ₙ-N=CR⁵R⁶, -(CR⁵R⁶)ₙ-CR⁵=NR⁵, -(CR⁵R⁶)ₙ-NHCOR⁵, -(CR⁵R⁶)ₙ-NHCOOR⁵, -SR⁵, -SO₂-R⁵, NR⁵-SO-R⁶, -SO-R⁵, des substituants hydrosolubilisants choisis parmi le groupe constitué par les radicaux carboxylate, sulfonate ou ammonium, COR⁵, COOR⁵, CONR⁵R⁶, CN, O=S(-R⁵)=O, O=S(-OR⁵)=O, O=S(-NR⁵R⁶)=O, R⁷OP(-OR⁸)=O, OAr, -(CR⁵R⁶)ₙ-Ar, -Hét, -NHHét, -OHét ou -(CR⁵R⁶)ₙ-Hét,
Sub² désigne Hal, hydroxyle, cyano, amino, nitro, C₁-C₄-alkyle ou C₁-C₄-alcoxy, COR⁵, COOR⁵, OAr, OHét, -(CR⁵R⁶)ₙ-Ar ou -(CR⁵R⁶)ₙ-Hét, -(CR⁵R⁶)ₙ-NR⁵R⁶, CONR⁵R⁶, CN, O=S(-R⁵)=O, O=S(-OR⁵)=O, O=S(-NR⁵R⁶)=O ou R⁷OP(-OR⁸)=O,
R⁷ et R⁸ désignent chacun, indépendamment l'un de l'autre, H, alkyle, alcoxy, alcényle, alcynyle, ayant chacun jusqu'à 20 atomes de C, cycloalkyle, cycloalcoxy, cycloalcényle, des systèmes bicycliques, ayant chacun jusqu'à 10 atomes de C, où ces radicaux peuvent être jusqu'à trisubstitués par Sub² et/ou un ou deux groupements CH₂ peuvent être remplacés par C=O et où les systèmes cycliques peuvent également contenir de 1 à 3 hétéroatomes choisis parmi N et/ou O,
pour la préparation de compositions pharmaceutiques destinées à la protection de la peau humaine ou des cheveux humains contre le rayonnement solaire, seuls ou en association avec des composés absorbant les UV connus en soi pour les compositions pharmaceutiques.

3. Utilisation de composés de formules Ik et/ou II, dans lesquelles les variables ont la signification selon la revendication 1 ou 2, comme stabilisateurs d'UV dans des compositions cosmétiques.

4. Composés issus du groupe
le N-(2-quinoxalinyl)-4-aminobenzoate d'isopropyle ;
l'acide N,N-benzoyl-(2-quinoxalinyl)-4-aminobenzoïque ;
l'acide N-(2-quinoxalinyl)-4-aminométhylbenzoïque ;
l'acide N-(2-quinoxalinyl)-2-amino-4,5-diméthoxybenzoïque ;
la N-(2-quinoxalinyl)-2-aminopyrimidine ;
la N-(2-quinoxalinyl)-2-aminobenzophénone ;
le N-(2-quinoxalinyl)-4-aminoanisole ;
la N-(2-quinoxalinyl)-3,4,5-triméthoxyaniline ;
la N-[bis(2-quinoxalinyl)]-2,4,6-trifluoroaniline ;
l'acide N-[2-(3-phényl)quinoxalinyl]-4-aminobenzoïque.

5. Composition photoprotectrice, comprenant des compositions cosmétiques ou pharmaceutiques destinées à la protection de l'épiderme humain ou des cheveux humains contre la lumière UV dans le domaine allant de 280 à 400 nm, **caractérisée en ce qu'**elle comprend, dans un véhicule cosmétiquement acceptable, seule ou conjointement avec des composés absorbant les UV connus en soi pour les compositions cosmétiques, comme filtres UV photostables, des quantités efficaces de composés de formules Ik et/ou II, dans lesquelles les variables ont la signification selon la revendication 1 ou 2.

6. Composition cosmétique ou pharmaceutique selon la revendication 5, dans laquelle la composition comprend un ou plusieurs filtres UV qui sont choisis de préférence parmi le groupe constitué par le 3-(4'-méthylbenzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, de sodium et de triéthanolamine de ceux-ci.

7. Composition cosmétique ou pharmaceutique selon la revendication 5 ou 6, **caractérisée en ce qu'**un ou plusieurs antioxydants et/ou au moins un parmi les acides pyrimidinecarboxyliques, l'ectoïne (acide (S)-1,4,5,6-tétrahydro-2-méthyl-4-pyrimidinecarboxylique) et l'hydroxyectoïne (acide (S,S)-1,4,5,6-tétrahydro-5-hydroxy-2-méthyl-4-pyrimidinecarboxylique) et/ou au moins un aryle oxime, préférablement le 2-hydroxy-5-méthyllaurophénone oxime, et/ou un dérivé de coumaranone, préférablement la 4,6,3',4'-tétrahydroxybenzyl-3-coumaranone ou le trisulfate de celle-ci, sont présents.
